(19) Europäisches Patentamt
European Patent Office
Office européen des brevets

(11) EP 2 489 348 B1

(12) **EUROPEAN PATENT SPECIFICATION**

(45) Date of publication and mention of the grant of the patent:
**26.11.2014 Bulletin 2014/48**

(21) Application number: **10820711.9**

(22) Date of filing: **01.10.2010**

(51) Int Cl.:
***A61K 9/14*** *(2006.01)* ***A61K 9/19*** *(2006.01)*
***A61K 31/7105*** *(2006.01)* ***A61K 31/711*** *(2006.01)*
***A61K 31/713*** *(2006.01)* ***A61K 31/715*** *(2006.01)*
***A61K 38/00*** *(2006.01)* ***A61K 38/28*** *(2006.01)*
***A61K 39/00*** *(2006.01)* ***A61K 39/395*** *(2006.01)*
***A61K 45/00*** *(2006.01)* ***A61K 47/02*** *(2006.01)*
***A61K 47/12*** *(2006.01)* ***A61K 48/00*** *(2006.01)*
***A61P 3/10*** *(2006.01)*

(86) International application number:
**PCT/JP2010/067241**

(87) International publication number:
**WO 2011/040597 (07.04.2011 Gazette 2011/14)**

(54) **PHARMACEUTICAL COMPOSITION CONTAINING MEDICAMENT-CONTAINING FINE PARTICLES AND METHOD FOR PRODUCING SAME**

PHARMAZEUTISCHE ZUSAMMENSETZUNG MIT MEDIKAMENTENHALTIGEN FEINEN PARTIKELN SOWIE HERSTELLUNGSVERFAHREN DAFÜR

COMPOSITION PHARMACEUTIQUE CONTENANT DE FINES PARTICULES RENFERMANT UN MEDICAMENT, ET PROCEDE DE PRODUCTION AFFERENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2009 JP 2009231001**

(43) Date of publication of application:
**22.08.2012 Bulletin 2012/34**

(73) Proprietor: **Nitto Denko Corporation**
**Osaka 567-8680 (JP)**

(72) Inventors:
- **OKUBO, Katsuyuki**
  **Ibaraki-shi**
  **Osaka 567-8680 (JP)**
- **OKAZAKI, Toshihiko**
  **Ibaraki-shi**
  **Osaka 567-8680 (JP)**
- **KITAURA, Chieko**
  **Ibaraki-shi**
  **Osaka 567-8680 (JP)**
- **MINOMI, Kenjiro**
  **Ibaraki-shi**
  **Osaka 567-8680 (JP)**
- **PEARSON, Elizabeth**
  **4002 Basel (CH)**
- **ROBERTS, Clive J.**
  **Nottingham NG7 2RD (GB)**
- **DAVIES, Martyn C.**
  **Nottingham NG7 2RD (GB)**
- **STOLNIK-TRENKIC, Snjezana**
  **Nottingham NG7 2RD (GB)**
- **ILLUM, Lisbeth**
  **Nottingham NG7 1BA (GB)**

(74) Representative: **Duckworth, Timothy John et al**
**J A Kemp**
**14 South Square**
**Gray's Inn**
**London WC1R 5JJ (GB)**

(56) References cited:
**WO-A1-2009/128357**
**WO-A1-2009/128357**
**JP-A- 63 208 522**
**JP-A- 2004 168 739**
**JP-A- 2004 501 188**
**JP-A- 2004 532 277**
**JP-A- 2005 126 335**
**JP-A- 2008 222 506**
**US-A1- 2005 170 005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 489 348 B1

## Description

Technical Field

The present invention relates to a pharmaceutical composition comprising a small particle comprised of a water-soluble drug and an ionic crystalline compound and a production method thereof.

Background Art

The advances of biotechnology have resulted in the discovery of a large number of therapeutic compounds such as peptides, proteins, polysaccharides, polynucleic acids, siRNAs, RNAs, antibodies, antigens and the like. The physicochemical characteristics of these compounds (e.g., large molecular weight, hydrophilicity, instability) make them difficult to administer into the body by other means than by injection. For some of these drugs a multiple daily dosing by injection is necessary and results in non-compliance especially among younger patients (non-patent documents 1 and 2).

When administered via an oral route or a transmucosal route such as pulmonary, transoral (oral mucosal), transvaginal and transnasal routes, and the like, these drugs are not easily absorbed across the mucosal surfaces due to their physical size and hydrophilicity. Furthermore, these drugs are prone to degradation by the enzymes such as peptidases and proteases, which is especially a problem in the gastrointestinal tract. In order to improve the transport of these drugs across mucosal surfaces, formulations containing absorption enhancers have been used with some success especially in delivery by the transnasal route and by the pulmonary route. However, there is a demand for the development of effective methods and compositions to provide the transport of high molecular weight compounds across mucosal surfaces.

Nanoparticles encapsulating drugs have been described as a mean of providing such transport (non-patent document 3). However, the encapsulation of peptides and proteins into nanoparticles is difficult due to the large size of these compounds and the normally hydrophobic environment in the matrix of a nanoparticle and results generally in a very low loading capacity and hence the need for large quantities of nanoparticles to be administered to the mucosal surface. This has to some extent been solved by the production of nanocrystals where the major part of the nanoparticle is pure drug. However, nanocrystal suspensions are generally very unstable and crystal growth normally takes place in a stepwise manner unless the crystal is stabilized. The normal procedure for stabilizing nanocrystals has been through a crosslinking procedure that to some extent crosslinks the drug itself hence lowering the bioactivity of the drug or rendering drugs such as proteins immunogenic. Furthermore, it is evident from publications in the literature that transport of nanoparticles across the mucosa is not readily achievable (non-patent document 3).

Nanoparticles have been widely investigated as carriers for drug delivery across the mucosa (non-patent documents 3-5). Nanoparticles have especially been of importance for peptides and proteins as outlined above. However, problems have been described in terms of lack of efficiency of the nanoparticle system to deliver sufficient quantities of drug across the mucosa, low drug-loading efficiency and compromised stability of the peptide and protein drugs if not encapsulated into the matrix of the nanoparticle. Nanoparticles in the form of nanocrystals of a peptide or a protein have been described in the literature as drug delivery systems for transmucosal and parenteral delivery (non-patent documents 6-7). However, the nanocrystals described in the literature are often physically unstable or if crosslinked to increase stability will lose some of the biological activity. Furthermore, often the nanocrystals have a size larger than 1 μm and hence are microcrystals rather than nanocrystals.

[Document List]

[patent documents]

patent document 1: US2004/0219224
patent document 2: WO98/46732
patent document 3: US7087246
patent document 4: WO02/072636
patent document 5: WO99/55310

[non-patent documents]

non-patent document 1: Drug Discovery Today. 7; 1184-1189 (2002)
non-patent document 2: J. Control. Rel. 87; 187-198 (2003)
non-patent document 3: J. Pharm. Sci. 96; 473-483 (2007)
non-patent document 4: Biomaterials 23; 3193-3201 (2002)
non-patent document 5: Int J Pharm. 342; 240-249 (2007)
non-patent document 6: J. Biotech. 113; 151-170 (2004)
non-patent document 7: Pharm. Res. 22(9); 1461-1470 (2005)

[SUMMARY OF THE INVENTION]

[Problems to be Solved by the Invention]

As mentioned above, encapsulation of a water-soluble polymer drug such as peptide, protein and the like in a small particle with high efficiency, and preparation of a small particle containing a water-soluble polymer drug in an appropriate size is not easy. In addition, it is not easy, when resuspending a composition containing a small dry particle in water when in use, to avoid aggregation and afford a redispersion of a particle having a small size.

It is an object of the present invention to provide a pharmaceutical composition that can be used for an efficient administration of a water-soluble drug by methods other than injection. More specifically, it is an object of the present invention to provide a small particle-containing pharmaceutical composition to be used for efficient transmucosal or transdermal administration of a water-soluble drug (e.g., peptide, protein, DNA, RNA, siRNA, polysaccharide, lipopeptide, lipoprotein, lipopolysaccharide, low-molecular-weight compound, antibody, antigen, toxin, vaccine and the like), particularly, a small particle-containing pharmaceutical composition which enables encapsulation of a water-soluble drug in a small particle with high efficiency and preparation of a small particle in an appropriate size, as compared to a conventional small particle-containing pharmaceutical composition. It is also an object of the present invention to provide a method for production of the pharmaceutical composition.

[Means of Solving the Problems]

The present inventors focused on transmucosal and transdermal administration using a small particle system such as nanoparticle as a method for efficiently administering the above-mentioned water-soluble drug by a method other than injection, and conducted diligent investigations. As a result, they have found that a small particle containing a water-soluble drug and an ionic crystalline compound, wherein the ionic crystalline compound is crystallized in the small particle, enables encapsulation of a water-soluble drug in a small particle with high efficiency and preparation of a small particle in an appropriate size, as compared to a conventional small particle-containing pharmaceutical composition. The present inventors have further found that coating of the surface of a small particle with a suitable polymer by electrostatic interaction enables stabilization of small particles in a suspension. Based on these findings, the present inventors found that a drug delivery system superior to conventional methods can be achieved by using a pharmaceutical composition containing the small particle, which resulted in the completion of the present invention.

Accordingly, the present invention is as follows.

[1] A pharmaceutical composition comprising a small particle comprised of (a) a water-soluble drug and (b) a pharmaceutically acceptable ionic crystalline compound which is solid at room temperature, and a pharmaceutically-acceptable polymer, wherein the ionic crystalline compound is crystallized in the small particle; and the small particle has a positive or negative charge at a predetermined pH, and the polymer has a charge of a sign opposite to that of the small particle at said pH, whereby the small particle and the polymer electrostatically interact with each other at said pH to form a complex wherein the polymer is attached to the surface of the small particle.

[2] The pharmaceutical composition of the above-mentioned [1], wherein the small particle is free of irreversible crosslinking.

[3] The pharmaceutical composition of the above-mentioned [1] or [2], wherein the small particle has an average particle size of not less than 10 nm and not more than 5000 nm.

[4] The pharmaceutical composition of any one of the above-mentioned [1] to [3], wherein the water-soluble drug is selected from the group consisting of peptide, protein, DNA, RNA, siRNA, polysaccharide, lipopeptide, lipoprotein, lipopolysaccharide, low-molecular-weight compound, antibody, antigen, toxin and vaccine.

[5] The pharmaceutical composition of any one of the above-mentioned [1] to [4], wherein the ionic crystalline compound comprises as constituent components, one or more types of cationic electrolytes selected from the group consisting of sodium ion, potassium ion, calcium ion, magnesium ion, zinc ion, iron ion and ammonium ion, and one or more types of anionic electrolytes selected from the group consisting of chlorine ion, sulfuric acid ion, lactic acid

ion, acetic acid ion, phosphoric acid ion, gluconic acid ion, carbonate ion and bicarbonate ion.

[6] The pharmaceutical composition of any one of the above-mentioned [1] to [5], wherein the water-soluble drug has a weight ratio of not less than 0.001 and not more than 100 relative to the ionic crystalline compound.

[7] The pharmaceutical composition of any one of [1] to [6], wherein the complex has an average particle size of not less than 15 nm and not more than 6000 nm.

[8] A method of producing the pharmaceutical composition of any one of the above-mentioned [1] to [7], comprising drying without heating an aqueous solution of the water-soluble drug and the ionic crystalline compound under reduced pressure to give the small particle and mixing a solution of the polymer with said pH and the small particle.

[9] The method of the above-mentioned [8], wherein the drying is freeze-drying or drying by centrifugal concentration.

[10] A production method of a pharmaceutical composition comprising a small particle comprised of (a) a water-soluble drug and (b) a pharmaceutically acceptable ionic crystalline compound which is solid at room temperature, the ionic crystalline compound being crystallized in the small particle, which method comprises drying without heating an aqueous solution of the water-soluble drug and the ionic crystalline compound under reduced pressure, optionally wherein the small particle is free of irreversible crosslinking.

[11] The method of above-mentioned [10], wherein the small particle has an average particle size of not less than 10 nm and not more than 5000 nm.

[12] The method of above-mentioned [10] or [11], wherein the water-soluble drug is selected from the group consisting of peptide, protein, DNA, RNA, siRNA, polysaccharide, lipopeptide, lipoprotein, lipopolysaccharide, low-molecular-weight compound, antibody, antigen, toxin and vaccine

[13] The method of any one of the above-mentioned [10] to [12], wherein the ionic crystalline compound comprises as constituent components, one or more types of cationic electrolytes selected from the group consisting of sodium ion, potassium ion, calcium ion, magnesium ion, zinc ion, iron ion and ammonium ion, and one or more types of anionic electrolytes selected from the group consisting of chlorine ion, sulfuric acid ion, lactic acid ion, acetic acid ion, phosphoric acid ion, gluconic acid ion, carbonate ion and bicarbonate ion.

[14] The method of any one of the above-mentioned [10] to [13], wherein the water-soluble drug has a weight ratio of not less than 0.001 and not more than 100 relative to the ionic crystalline compound.

[15] The method of any one of the above-mentioned [10] to [14], wherein the drying is freeze-drying or drying by centrifugal concentration.

[Effect of the Invention]

The pharmaceutical composition of the present invention enables efficient transmucosal or transdermal administration of a water-soluble drug (e.g., peptide, protein, DNA, RNA, siRNA, polysaccharide, lipopeptide, lipoprotein, lipopolysaccharide, low-molecular compound, antibody, antigen, toxin and vaccine and the like) to a mammal, since a small particle contained in the pharmaceutical composition has an appropriate particle size and can concentrate the water-soluble drug in the small particle.

Moreover, in the pharmaceutical composition of the present invention comprising a complex wherein the surface of a small particle is coated with a polymer, the complex has high stability (e.g., preservation stability, stability against enzymes, and the like), and permits adjustment of a drug release rate according to the preparation conditions and the kind of the polymer. Therefore, the release property desirable for sustained-release preparation and vaccine can be realized.

According to the production method of the present invention, moreover, a water-soluble drug (e.g., peptide, protein, DNA, RNA, siRNA, polysaccharide, lipopeptide, lipoprotein, lipopolysaccharide, low-molecular-weight compound, antibody, antigen, toxin and vaccine and the like) can be encapsulated in a small particle in a high yield. Moreover, according to the production method of the present invention, a dried small particle free of moisture by freeze-drying and the like can be obtained easily, whereby the preservation stability of a water-soluble drug can be ensured.

[Brief Description Of The Drawings]

Fig. 1 shows the results of the particle size measurement in Example 1, wherein the horizontal axis shows the particle size (nm), the vertical axis shows the scattering intensity distribution (index value), and the particle size is shown by the particle diameter.

Fig. 2 shows the results of the particle size measurement in Example 2, wherein the horizontal axis shows the particle size (nm), the vertical axis shows the scattering intensity distribution (index value), and the particle size is shown by the particle diameter.

Fig. 3 shows the results of the particle size measurement in Comparative Example 1, wherein the horizontal axis

shows the particle size (nm), the vertical axis shows the scattering intensity distribution (index value), and the particle size is shown by the particle radius.

Fig. 4 shows the results of the particle size measurement in Example 3, wherein the horizontal axis shows the particle size (nm), the vertical axis shows the scattering intensity distribution (index value), and the particle size is shown by the particle radius.

Fig. 5 shows the results of the particle size measurement in Comparative Example 2, wherein the horizontal axis shows the particle size (nm), the vertical axis shows the scattering intensity distribution (index value), and the particle size is shown by the particle diameter.

Fig. 6 shows the results of the particle size measurement in Example 4, wherein the horizontal axis shows the particle size (nm), the vertical axis shows the scattering intensity distribution (index value), and the particle size is shown by the particle diameter.

Fig. 7 shows the results of the particle size measurement in Example 5, wherein the horizontal axis shows the particle size (nm), the vertical axis shows the scattering intensity distribution (index value), and the particle size is shown by the particle diameter.

Fig. 8 shows the results of the particle size measurement in Example 6, wherein the horizontal axis shows the particle size (nm), the vertical axis shows the scattering intensity distribution (index value), and the particle size is shown by the particle radius.

Fig. 9 shows the results of the particle size measurement in Example 7, wherein the horizontal axis shows the particle size (nm), the vertical axis shows the scattering intensity distribution (index value), and the particle size is shown by the particle radius.

Fig. 10 shows the results of the particle size measurement in Example 8, wherein the horizontal axis shows the particle size (nm), the vertical axis shows the scattering intensity distribution (index value), and the particle size is shown by the particle radius.

Fig. 11 shows the results of the particle size measurement in Example 9, wherein the horizontal axis shows the particle size (nm), the vertical axis shows the scattering intensity distribution (index value), and the particle size is shown by the particle diameter.

Fig. 12 shows the results of the particle size measurement in Example 10, wherein the horizontal axis shows the particle size (nm), the vertical axis shows the scattering intensity distribution (index value), and the particle size is shown by the particle diameter.

Fig. 13 shows the results of the particle size measurement in Example 11, wherein the horizontal axis shows the particle size (nm), the vertical axis shows the scattering intensity distribution (index value), and the particle size is shown by the particle diameter.

Fig. 14 shows the results of the particle size measurement in Comparative Example 3, wherein the horizontal axis shows the particle size (nm), the vertical axis shows the scattering intensity distribution (index value), and the particle size is shown by the particle radius.

Fig. 15 shows the results of the particle size measurement in Comparative Example 4, wherein the horizontal axis shows the particle size (nm), the vertical axis shows the scattering intensity distribution (index value), and the particle size is shown by the particle radius.

Fig. 16 shows a TEM image of the small particle of Example 10.

Fig. 17 shows the results of the elemental analysis of the small particles in the TEM image of Example 10.

Fig. 18 shows a TEM image of the small particle of Example 11.

Fig. 19 shows the results of the elemental analysis of the small particles in the TEM image of Example 11.

Fig. 20 shows the results of the stability against enzyme test of chitosan-coated insulin small particle in Experimental Example 4, wherein the horizontal axis shows the percentage of the insulin content after shaking reaction at 37°C for 30 min relative to the initial content, from top to bottom in the order of i) a mixed sample of a surface-coated small particle and an enzyme, ii) a mixed sample of a surface-coated small particle and a buffer, iii) a mixed sample of an insulin solution and an enzyme, and iv) a mixed sample of an insulin solution and a buffer.

[Embodiment of the Invention]

The present invention provides a pharmaceutical composition comprising a small particle comprised of (a) a water-soluble drug and (b) a pharmaceutically acceptable ionic crystalline compound which is solid at room temperature, and a pharmaceutically acceptable polymer, wherein the ionic crystalline compound is crystallized in the small particle; and the small particle has a positive or negative charge at a predetermined pH, and the polymer has a charge of a sign opposite to that of the small particle at said pH, whereby the small particle and the polymer electrostatically interact with each other at said pH to form a complex wherein the polymer is attached to the surface of the small particle.

The pharmaceutical composition can be used for, for example, transmucosal or transdermal administration. Accordingly, the pharmaceutical composition of the present invention can be administered to the mucosa (e.g., mucosa

such as that in the nose, mouth cavity, eye, vagina and gastrointestinal tract) or skin of a subject in need of treatment and/or therapy by an appropriate method such as coating, spraying, nebulizing, patching and the like, whereby a drug can be delivered to the mucosa or skin tissue, or circulatory or immunity system via mucosa or skin tissue to provide efficacy, vaccine effect and the like.

The above-mentioned "water-soluble drug" is appropriately selected according to the intended use of a pharmaceutical composition. The drug may be crystalline or noncrystalline. In the present specification, "water-soluble" means compatibility with water at ambient temperature. The choice of the drug is not particularly limited as long as the drug shows a minimal solubility in water at ambient temperature. More specifically, while the water-soluble drug usable for a pharmaceutical composition of the present invention is not limited to the following, for example, it is preferably dissolved in water (100 g) at 25°C in an amount of not less than 0.0001 g, more preferably not less than 0.001 g, still more preferably not less than 0.01 g. As a solvent in which the drug is dissolved, a mixture of an organic solvent (ethanol and the like) and water, an aqueous acid solution and aqueous alkali solution can be used, in addition to simple water, and an appropriate solvent can be appropriately selected according to the chemical property of the drug. As the drug, a drug that can be dissolved in an organic solvent that can be mixed with water, such as methanol, ethanol, isopropanol, acetone, acetonitrile and the like, or a drug that can be dissolved in a mixture of such organic solvent and water is preferable.

The molecular weight of the drug is not particularly limited, and may be a low-molecular-weight compound or a polymer compound. The low-molecular-weight compound in the present specification means a compound having a molecular weight of less than 500D, and the polymer compound in the present specification means a compound having a molecular weight of not less than 500D. Examples of the drug include peptide, protein, DNA, RNA, siRNA, polysaccharide, lipopeptide, lipoprotein, lipopolysaccharide, low-molecular-weight compound, antibody, antigen, toxin and vaccine and the like. Examples of the drug also include various peptides, proteins, DNAs, RNAs, siRNAs, polysaccharides, lipopeptides, lipoproteins, lipopolysaccharides, low-molecular-weight compounds, antibodies, antigens, toxins, vaccines and the like such as antihypertensive agent, antihypotensive agent, analgesic, antipsychotic agent, antidepressant, antimanic, antianxiety agent, sedative, hypnotic, antiepileptic, opioid agonist, therapeutic agent for asthma, anesthetic, antiarrhythmic agent, therapeutic agent for arthritis, antiepileptic drugs, ACE inhibitor, decongestant, antibiotic, antianginal agent, diuretic, antiparkinson agent, bronchodilator, oxytocic, antidiuretic, antilipemic agent, immunosuppressant, immunity regulator, antiemetic, antiinfective agent, antineoplastic, antifungal agent, antivirus agent, antidiabetic agent, antiallergic agent, fever reducer, antitumor agent, antigout agent, antihistamine agent, antipruritic agent, bone regulator, cardiovascular agent, hypocholesterolemic agent, antimalarial agent, pharmaceutical agent for ceasing smoking, antitussive agent, expectorant, mucolytic agent, nasal decongestant, dopamine agonist, pharmaceutical agent for digestive tract, muscle relaxant, neuromuscular blocker, parasympatholytic, prostaglandin, stimulant drug, anorectic agent, thyroid agent or antithyroid agent, hormone, antimigraine agent, antiobestic agent, antiinflammatory agent and the like.

Specific examples of the drug include, but are not limited to, insulin, glucagon, leuprolide, growth hormones, parathyroid hormones, calcitonin, vascular endothelial growth factor, erythropoietin, heparin, cyclosporin, oxytocin, tyrosine, enkephalin, tyrotropin releasing hormone, follicle-stimulating hormone, leuteinising hormone, vasopressin, vasopressin analogs, catalase, superoxide dismutase, interleukin II, interferons, colony stimulating factor, tumor necrosis factor, melanocyte stimulating hormone, glucagon-like peptide-1, glucagon-like peptide-2, katacalcin, cholecystekinin-12, cholecystekinin-8, exendin, gonadoliberin-related peptide, insulin-like protein, leucine-enkephalin, methionine-enkephalin, leumorphine, neurophysin, copeptin, neuropeptide Y, neuropeptide AF, PACAP-related peptide, pancreatic hormone, peptide YY, urotensin, intestinal peptide, adrenocorticotropic peptide, epidermal growth factor, prolactin, luteinising hormone releasing hormone (LHRH), LHRH agonist, growth hormone releasing factor, somatostatin, gastrin, tetragastrin, pentagastrin, endorphins, angiotensins, tyrotropin releasing hormone, granulocyte-colony stimulating factor, granulocyte-macrophage-colony stimulating factor, heparinase, antigens for influenza vaccine, tetanus toxins, diphtheria toxin, cancer antigen protein, cancer antigen peptide, β-amyloid, immunoglobulins, siRNAs for treatment of cirrhosis, siRNAs for treatment of cancer, low-molecular compounds such as bromhexine, granisetron, zolmitriptan, sumatriptan and the like, and pharmaceutically acceptable salts thereof, and the like. It is also possible to appropriately use two or more kinds of drugs in combination.

The choice of the above-mentioned "pharmaceutically acceptable ionic crystalline compound which is solid at room temperature" is not particularly limited as long as it is an ionic crystalline compound which is solid at room temperature and pharmaceutically acceptable. A compound, having a melting point, decomposition temperature, or sublimation temperature of not less than 40°C is preferable, not less than 100°C is more preferable, and not less than 200°C is still more preferable. A compound having a melting point, decomposition temperature, or sublimation temperature of less than 40°C is not preferable since it cannot provide a stable solid crystal in a small particle, and shows low ability to precipitate or form a particle of a water-soluble drug. While the upper limit of the melting point, decomposition temperature, or sublimation temperature of the compound is not particularly limited, the melting point, decomposition temperature, or sublimation temperature is preferably not more than 2000°C, more preferably not more than 1800°C, still more preferably not more than 1500°C.

As mentioned above, the ionic crystalline compound is crystallized in small particles. The "crystallization" here is not particularly limited as long as crystals of the ionic crystalline compound are dispersed inside the small particle comprised of the water soluble compound and the ionic crystalline compound when the small particle is observed by, for example, a preferable apparatus such as a transmission electron microscope and the like. As shown in the below-mentioned Examples, whether the crystal is derived from the compound can be confirmed by elemental analysis of crystals by, for example, XMA. It is considered that a salt is locally contained at high concentration by crystallization of ionic crystalline compound in small particles, which enables efficient precipitation of a water-soluble drug in the area surrounding the salt and particle formation, as well as inhibits aggregation of the drug to prevent small particles from having a greater size.

The ionic crystalline compound usable for the pharmaceutical composition of the present invention includes, but is not limited to, for example, an ionic crystalline compound comprising, as constituent components, one or more kinds of cationic electrolytes selected from the group consisting of sodium ion, potassium ion, calcium ion, magnesium ion, zinc ion, iron ion and ammonium ion, and one or more kinds of anionic electrolyte selected from the group consisting of chlorine ion, sulfuric acid ion, lactic acid ion, acetic acid ion, phosphoric acid ion, gluconic acid ion, carbonate ion and bicarbonate ion. Among those, examples of preferable ionic crystalline compound include sodium chloride, potassium chloride, ammonium sulfate and sodium sulfate, since they are highly safe to the body and show good reproducibility of drug precipitation due to high regularity of crystal shape of the salt.

While the weight ratio of the water-soluble drug to the ionic crystalline compound in the pharmaceutical composition of the present invention is not particularly limited as long as a small particle with preferable characteristics that meets a desired object can be obtained, it is typically not less than 0.001 and not more than 100, preferably not less than 0.005 and not more than 50, more preferably not less than 0.01 and not more than 10. By selecting such weight ratios, efficient drug precipitation and formation of salt crystal can be realized.

The water content of the small particle varies depending on the state of the surrounding area. For example, it is negligible in a dry state, but water is considered to penetrate somewhat into the small particle in water. Since the water content of the small particle in water is essentially irrelevant to the structure and property of the small particle, the range of the water content of the small particle is also not particularly limited. For example, it is 0.01 - 99.99 wt%.

Moreover, the small particle is preferably free of irreversible crosslinking such as covalent bonds, from the aspects of physiological activity, immunogenicity and the like, as mentioned above. The irreversible crosslinking here does not include pseudo crosslinking (e.g., electrostatic interaction) that can be reversibly dissociated by addition of water.

The size of the small particle in the pharmaceutical composition of the present invention is preferably an average particle size of 10 - 5000 nm, more preferably 20 - 4000 nm, still more preferably 50 - 3000 nm. A size of the small particle of greater than 5000 nm is not preferable, since even when the same amount of a drug is administered, the number of small particles decreases, the total surface area of the small particles reduces and the drug release rate becomes low. It is also undesirable since the efficiency of uptake by immunocompetent cells decreases when used as a particle for vaccine. In addition, a size of the small particle of smaller than 10 nm is not preferable, since the amount of a drug contained per one small particle becomes too small, and the pharmacological effect or vaccine effect after administration becomes weak. It is also undesirable since small particles easily aggregate during production or use, and the handling becomes difficult.

The particle size here refers to a value obtained by measuring small particles dispersed in water, which is a diameter calculated on the assumption that the small particles have a spherical shape. To be specific, the particle size is measured by a dynamic light scattering measuring apparatus, and an average of the hydrodynamic diameter determined from the scattering intensity distribution is employed as an average particle size. Here, an average particle size of small particles of, for example, not less than 10 nm means that not less than 10%, preferably not less than 20%, more preferably not less than 30%, still more preferably not less than 40%, particularly preferably not less than 50% of the average particle size of peaks is not less than 10 nm in the proportion of each particle size peak in the above-mentioned scattering intensity distribution by the dynamic light scattering measuring apparatus (proportion of cumulative scattering intensity for each particle size peak to cumulative scattering intensity for all peaks).

The pharmaceutical composition of the present invention contains a pharmaceutically acceptable polymer. The small particles are constituted to have a positive or negative charge at a predetermined pH, and a polymer having a charge of the sign opposite to that of the small particle at said pH is used as the polymer, whereby they are allowed to electrostatically interact with each other to form a complex having the polymer attached to the surface of the small particle.

As long as the small particle has a positive or negative charge at a predetermined pH, the aforementioned water-soluble drug, ionic crystalline compound and small particle can also be used for this embodiment.

In the following, when a polymer is attached to the surface of the small particle, it is sometimes expressed that a small particle is "coated" with the polymer and the like, and a complex wherein a polymer is attached to the surface of the small particle, it is sometimes referred to as a "surface-coated small particle". Furthermore, a small particle free of coating with a polymer is sometimes referred to as a "small core particle" or simply as a "core particle", so as to expressly distinguish a surface-coated small particle. Coating of a small core particle with a polymer is not particularly

limited as long as a polymer is attached to the surface of a small core particle by an electrostatic interaction between the small particle and the polymer.

The pharmaceutically acceptable polymer making up the surface-coated small particle (hereinafter to be referred to as a "surface-coating polymer") is preferably biocompatible. The term "biocompatibility" herein means that a substance and decomposed products thereof have no toxic or hazardous effect on a body tissue or a body system (e.g., blood circulation system, nerve system, immune system and the like) and cause no undue immune rejection. The biocompatible polymer is suitable for administration to human or other animals. In addition, the polymer is more preferably biodegradable. The term "biodegradability" herein means that a substance is decomposed within a living body by an enzymatic, chemical or physical process or the like within an acceptable period of time to form a smaller chemical species. Methods for examining biocompatibility and biodegradability of a substance are well known in the technical field of the invention.

The surface-coating polymer may be a natural polymer or a synthetic polymer. Since the surface-coating polymer electrostatically interacts with the small particle on the surface of the small particle at the predetermined pH, it needs to be a polymer having a charge of the sign opposite to that of the small particle at the pH. Where necessary, more than one polymer can be used in combination for the surface-coating polymers as long as they are capable of having charges of the same sign at the pH.

The pharmaceutical composition of the present invention comprising the surface-coated small particle can be produced, for example, by the below-mentioned production method; when the composition is produced by the production method, even if the surface-coating polymer is by itself slightly water-soluble at the predetermined pH, the composition can be produced by mixing the surface-coating polymer with the small particle at a pH where the surface-coating polymer is readily water-soluble, and then adjusting the pH of the mixture to the predetermined pH. Therefore, as the surface-coating polymer, not only polymers that are readily water-soluble at the predetermined pH, but also polymers that are slightly water-soluble at the predetermined pH can be used.

As the polymer that can be used for the surface-coating polymer, polyanionic or polycationic polysaccharides, polyamino acids and other charged polymers can be mentioned without limit. The polymer is appropriately selected based on the kind of the drug used, the charge, and the like.

Polyanionic polysaccharides that can be used in the present invention means a polysaccharide that has one or more acidic polar groups such as a carboxyl group, a sulfuric acid group or a phosphoric acid group in the constitutional unit. Examples of such polyanionic polysaccharides include, but are not limited to, chondroitin sulfuric acid, dextran sulfuric acid, carboxymethylcellulose, alginic acid, pectin, hyaluronic acid, derivatives and salts thereof and the like.

Polycationic polysaccharides that can be used in the present invention means a polysaccharide that has one or more basic polar group such as an amino group in the constitutional unit. Examples of such polycationic polysaccharides include, but are not limited to, chitin, chitosan, derivatives and salts thereof and the like. Chitosan and chitosan derivative can be selected from those having various molecular weights and degrees of deacetylation, and chitosan derivative can be selected from those having various degrees of substitution.

The polyanionic polyamino acid that can be used in the present invention means a polyamino acid whose isoelectric point is on the acidic side of the physiological pH; examples thereof include, but are not limited to, polyglutamic acid, polyaspartic acid, derivatives and salts thereof and the like.

The polycationic polyamino acid that can be used in the present invention means a polyamino acid whose isoelectric point is on the basic side of the physiological pH; examples thereof include, but are not limited to, polylysine, polyarginine, derivatives and salts thereof and the like.

Examples of the polymer that can be used for the surface-coating polymer other than the above-mentioned polysaccharides and polyamino acids, include polyethylenimine, polyacryl acid, derivatives and salts thereof and the like.

The surface-coating polymer may be polyethylene glycolated (PEGylated) and/or glycosylated.

The surface-coating polymer may be mucoadhesive, and/or act as a transmucosal absorption promoter. Examples of mucoadhesive polymers include chitosan, polyacryl acid, sodium alginate, carboxymethylcellulose and the like as well as PEGylated polymers thereof and the like. Examples of the polymers that functions as transmucosal absorption promoters include chitosan, polyacryl acid, polyarginine, salts and derivatives thereof and the like.

Those ordinarily skilled in the art can determine the molecular weight of a surface-coating polymer in consideration of factors such as degradation rate, mechanical strength, solubility, which kind of small particle would form the complex with the surface-coating polymer, and the like. Typically, the weight average molecular weight of the surface-coating polymer should preferably be not less than 1,000 Da, and more preferably not less than 2,000 Da; preferably not more than 1,000,000 Da, and more preferably not more than 500,000 Da, as measured by gel permeation chromatography. Accordingly, typically, the weight average molecular weight of the surface-coating polymer is preferably between 1,000-1,000,000 Da, and more preferably between 2,000-500,000Da. For example, the weight average molecular weight of chitin or chitosan may be between 1,000-1,000,000 Da. and the degree of deacetylation of chitin or chitosan may be between 20-100%.

The size of the surface-coated small particle is, for example, an average particle size of preferably 15 - 6000 nm, more preferably 25 - 5000 nm, still more preferably 55 - 4000 nm. When the surface-coated small particle size is

greater than 6000 nm, the efficiency of uptake by immunocompetent cells unpreferably decreases when used as a small particle for vaccine. A surface-coated small particle size smaller than 15 nm is not preferable, since small particles easily aggregate during production or use, and the handling becomes difficult.

The particle size here refers to a value obtained by measuring surface-coated small particles dispersed in water, and a diameter calculated on the assumption that the surface-coated small particles have a spherical shape. Specifically, the particle size is measured by a dynamic light scattering measuring apparatus, and an average of the hydrodynamic diameter determined from the scattering intensity distribution is employed as an average particle size. Here, an average particle size of surface-coated small particles of, for example, not less than 15 nm means that not less than 10%, preferably not less than 20%, more preferably not less than 30%, still more preferably not less than 40%, particularly preferably not less than 50% of the average particle size of peaks is not less than 15 nm in the proportion of each particle size peak in the above-mentioned scattering intensity distribution by the dynamic light scattering measuring apparatus (proportion of cumulative scattering intensity for each particle size peak to cumulative scattering intensity for all peaks).

As the particle size of small core particle in surface-coated small particles, a particle size observed by a microscope such as TEM, SEM, AFM and the like may be employed.

The preferable relative proportions of the drug, the ionic crystalline compound and the surface-coating polymer in the pharmaceutical composition of the present invention comprising the surface-coated small particle vary depending on the kind of the drug, ionic crystalline compound and surface-coating polymer to be used, the size of the small core particle and the like and hence cannot be stated in general. The relative proportion as expressed by the weight ratio in a pharmaceutical composition may be, for example, drug:ionic crystalline compound:surface-coating polymer=1:0.01 - 1000:0.1 - 1000.

The predetermined pH is desirably set to a suitable physiological pH of the administration site so as to avoid topical irritation that occurs when the pharmaceutical composition of the present invention is administered to the body. As mentioned above, the pharmaceutical composition of the present invention can be administered to mucosa such as that in the lung, mouth cavity, eye, vagina, intestine, nose and the like or skin, where the physiological pH varies in these various mucosas or skin. For example, the physiological pH of the gastrointestinal tract increases along the length thereof from about pH 1 in the stomach to pH 8 in the colon; the mouth cavity has a pH around 6.8; the pH of nasal fluid is within the range of about pH 5.5 to 6.5; the pH of vagina is around 4.5. For example, when the pharmaceutical composition of the present invention is to be administered to the nasal mucosa, preferable pH value of the composition is, for example, about 6.0.

The pharmaceutical composition of the present invention may be incorporated into any dosage form as long as the small particle (i.e., small core particle or surface-coated small particle) can directly reach the target site. Examples thereof include pulmonary agent, oral agent, buccal agent, intraocular agent, vaginal agent, intranasal agent, suppository, percutaneous absorber and the like.

As the pulmonary agent, an inhalant which is delivered to alveoli by some lung inhaler is preferred.

As the oral agent, usual oral preparations, for example, tablet, granule, fine granule, capsule and the like can be mentioned. Dosage forms designed to release the drug in the small intestine, for example, enteric tablet, enteric granule, enteric capsule and enteric fine granule are preferred.

As the buccal agent, the intraocular agent and the intranasal agent, buccal tablet, buccal spray, eye drop, nasal drop, aerosol, ointment, gel, cream, liquid, suspension, lotion, dry powder, sheet, patch and the like can be mentioned.

As the vaginal agent and suppository, ointment, gel, cream, liquid, suspension, lotion, dry powder, sheet, capsule and the like can be mentioned.

The transdermal absorbent is not particularly limited and may be in any dosage form as long as the drug can be absorbed from the skin. For example, ointment, gel, cream, gel cream, liquid, lotion, aerosol, liniment, plaster, adhesive skin patch, reservoir patch and the like can be mentioned. These dosage forms may be used alone and noninvasively, or in a combination with an invasive device such as microneedle, stratum corneum peeling tape, ablation and the like.

As a method for preparing the above-mentioned dosage forms, known production methods generally used in the field can be applied. Where necessary, when preparing the above-mentioned dosage forms, carriers such as excipient, binder, disintegrant and lubricant, and various preparation additives such as sweetening agent, surfactant, suspending agent, emulsifier, colorant, preservative and stabilizer, which are generally used for preparing the particular dosage forms, can be appropriately added in an appropriate quantity to produce the dosage forms. Also, the pharmaceutical composition of the present invention can be preserved in the form of a dry powder prepared by lyophilizing the suspension, and the like, and resuspended by adding water to the dry powder when in use. Employing this method, hydrolysis can be avoided to improve the preservation stability of the pharmaceutical composition.

The ratio of the small particle in the pharmaceutical composition of the present invention (i.e., small core particle or surface-coated small particle) is preferably 0.01 - 100 wt%, more preferably 0.1 - 100 wt%.

The pharmaceutical composition of the present invention is stable and with low toxicity, and can be used safely. The administration frequency and single dose vary depending on the drug used, condition and body weight of patient,

administration route, therapeutic strategy and the like and hence cannot be stated in general. For example, when the composition of the present invention in which insulin is used as the drug, is transnasally administered to a patient with diabetes and the like, as one therapeutic strategy, about 2 to about 6 mg of the active ingredient (insulin) can be administered to an adult (about 60 kg in body weight) before each meal.

The present invention also provides a production method of the aforementioned pharmaceutical composition. It should be noted that the pharmaceutical composition of the present invention can also be produced by a production method other than the method explained below.

The above-mentioned small core particle can be prepared by a method comprising drying an aqueous solution of the above-mentioned water-soluble drug and the above-mentioned ionic crystalline compound under reduced pressure without heating.

The concentration of a drug in an aqueous solution containing a drug and an ionic crystalline compound is preferably 0.01 - 30 wt%, more preferably 0.05 - 20 wt%. When the drug concentration is lower than 0.01 wt%, the composition ratio of the drug in a pharmaceutical composition to be produced becomes unpreferably small, and the production yield becomes unpreferably low. A drug concentration higher than 30 wt% is not preferable, since the particle size of particles obtained by drying cannot be made small.

The concentration of an ionic crystalline compound in an aqueous solution containing a drug and an ionic crystalline compound is preferably 0.1 - 50 wt%, more preferably 0.5 - 30 wt%. A concentration of the ionic crystalline compound of lower than 0.1 wt% is not preferable, since drug precipitation and particle formation cannot be efficiently performed. A concentration of the ionic crystalline compound of higher than 50 wt% is not preferable, since drug precipitation starts before drying and small core particles having a small size cannot be produced.

As mentioned above, in this method, drying is performed under reduced pressure without heating. The "drying" here means an operation to remove water from the above-mentioned aqueous solution substantially completely or partially.

Drying under reduced pressure is performed under a pressure of preferably 0 - 10000 Pa, more preferably 0 - 600 Pa, still more preferably 0 - 200 Pa. A pressure higher than 10000 Pa is not preferable since water cannot be removed efficiently.

In this method, the drying without heating is performed under a temperature condition of preferably -200°C to 40°C, more preferably -100°C to 35°C. Drying at a temperature of higher than 40°C is not preferable since the drug solubility is improved, and the speed of drug precipitation and particle formation by drying operation becomes slow. As a result, the particle size becomes large.

For the above-mentioned drying, freeze-drying or centrifugal concentration drying can be utilized.

The freeze-drying in this production method comprises rapidly cooling to freeze the above-mentioned aqueous solution of a drug and an ionic crystalline compound using a freezer or liquid nitrogen, promptly setting the frozen product on a freeze-dryer and removing without heating water under reduced pressure. By this operation, the above-mentioned small core particle can be prepared. As a freeze-dryer, any types of dryers used in the art can be used appropriately. For example, freeze dryer DC400 of Yamato Scientific Co., Ltd. can be mentioned. In a production method using freeze-drying, the above-mentioned aqueous solution is frozen by rapidly cooling using a freezer or liquid nitrogen, whereby the drug can be rapidly insolubilized and precipitated. As a result, a small core particle having a smaller particle size can be preferably produced. It is also preferable since scaled-up production is possible by utilizing freeze-drying.

In the drying by centrifugal concentration in the production method, the above-mentioned aqueous solution of a drug and an ionic crystalline compound is treated by centrifugation using a suitable centrifugal concentration dryer and dried without heating under reduced pressure to remove water. By this operation, the above-mentioned small core particles can be prepared. As the centrifugal concentration dryer, any type used in the art can be appropriately used. For example, Jouan concentrator evaporator system (RC10.22) and Concentrator 5301 manufactured by Eppendorf can be mentioned. Since the production method using centrifugal concentration drying permits drug precipitation and particle formation by removing water at ambient temperature, it can be utilized as an alternative means to avoid freeze-drying when the stability of the drug is influenced by a freeze-drying operation and the like.

The above-mentioned surface-coated small particles can be prepared by a method including mixing small core particles obtained as mentioned above with a solution of a surface-coating polymer having the above-mentioned predetermined pH. When the surface-coating polymer is poorly soluble at said pH, a surface-coated small particle can be prepared by a method including dissolving the surface-coating polymer in a solution at pH at which the surface-coating polymer is readily soluble, adding the small core particle, and adjusting the pH of the mixture to the predetermined pH. By these methods, a suspension of the surface-coated small particles can be obtained. The obtained suspension may or may not be dialyzed.

A suspension of small core particles or surface-coated small particles obtained by the aforementioned method is formulated into the above-mentioned suitable dosage form by a known production method generally used in the technical field. As mentioned above, where necessary, when the above-mentioned dosage form is prepared, a carrier such as excipient, binder, disintegrant and lubricant, and various preparation additives such as sweetening agents,

surfactants, suspending agents, emulsifiers, colorants, preservatives and stabilizers, which are generally used for preparing the particular dosage form can be appropriately added in an appropriate quantity to produce the dosage forms. Also, the pharmaceutical composition of the present invention can be produced in the form of a dry powder prepared by lyophilizing the suspension, and the like.

While the present invention is hereinafter further explained in detail by referring to Examples, Comparative Examples and Experimental Examples, the present invention is not limited by the following Examples and the like.

[Examples]

In the following Examples and Comparative Examples, the particle size of particles in a suspension was measured by a dynamic light scattering method using DLS 802 (Viscotek) or ELS-8000 (Otsuka Electronics Co., Ltd.). The zeta potential of the particle of each sample was measured using Zeta sizer nano (Malvern).

Example 1: preparation of uncoated insulin core particle by centrifugal concentration drying

In this Example, insulin (isoelectric point (pI) = 5.3) was used as a protein drug, and insulin core particles (uncoated) were prepared by centrifugal concentration to remove water from an insulin solution containing an ionic crystalline compound to allow precipitation of insulin.

The preparation included the following steps.

Hydrochloric acid (0.02 M) (3 ml) was added to dissolve Bovine Insulin (Sigma) (30 mg), and 2 ml of a salt solution (trisodium citrate dihydrate 58 mg/ml, zinc chloride 1.4 mg/ml, sodium chloride 180 mg/ml) was added. Thereafter, NaOH (0.1 M) was added to adjust the pH to 6.25. The obtained solution (0.25 ml) was transferred to a glass vial, and water was removed without heating by centrifugal concentrator under reduced pressure to dry the solution. Water (2 ml) was added to the dried product to give a suspension for particle size measurement.

The size of the particles in the obtained suspension was measured. As a result, the diameter of the particles in the main resultant product was 730 nm (Fig. 1). Accordingly, it was found that core particles having a small particle size can be obtained by the above-mentioned preparation method.

In addition, the zeta potential of the particles in the obtained suspension was measured. As a result, the zeta potential was -10 mV at pH 6. Reflecting the minus charge of insulin at pH 6, the core particles were also charged minus.

Example 2: preparation of uncoated insulin core particle by freeze-drying

In this Example, insulin (pI=5.3) was used as a protein drug, and insulin core particles (uncoated) were prepared by freeze-drying to remove water from an insulin solution containing an ionic crystalline compound to allow precipitation of insulin.

The preparation included the following steps.

Hydrochloric acid (0.02 M) (3 ml) was added to dissolve Bovine Insulin (Sigma) (30 mg), and 2 ml of a salt solution (trisodium citrate dihydrate 58 mg/ml, zinc chloride 1.4 mg/ml, sodium chloride 180 mg/ml) was added. Thereafter, NaOH (0.1 M) was added to adjust the pH to 6.25. The obtained solution (0.25 ml) was transferred to a glass vial, frozen with liquid nitrogen and water was removed without heating by a freeze drier under reduced pressure to dry the solution. Water (2 ml) was added to the dried product to give a suspension for particle size measurement.

The size of the particles in the obtained suspension was measured. As a result, the diameter of the particles in the main resultant product was 180 nm (Fig. 2). Accordingly, it was found that core particles having a small particle size can also be obtained by the above-mentioned preparation method.

Comparative Example 1: preparation of uncoated insulin core particles by cooling

In this Comparative Example, insulin (pI=5.3) was used as a protein drug, and preparation of insulin core particles (uncoated) was tried by cooling an insulin solution containing an ionic crystalline compound to allow precipitation of insulin.

The preparation included the following steps.

Hydrochloric acid (0.02 M) (3 ml) was added to dissolve Bovine Insulin (Sigma) (30 mg), and 2 ml of sodium citrate (0.2 M), 24 μl of zinc chloride solution (0.12 g/ml), and 0.36 g of sodium chloride were added and mixed therewith. Thereafter, a small amount of NaOH (0.1 M) was added to adjust the pH to 6.25. The obtained solution (1 ml) was transferred to a glass vial and cooled overnight at 4°C to allow crystal precipitation, whereby an insulin particle suspension was obtained.

The size of the particles in the obtained suspension was measured. As a result, the particles diameter varied greatly from 6 nm considered to be a gathering of several molecules of insulin to a large aggregate, showing insufficient

control of the particle size (Fig. 3).

Example 3: preparation of uncoated insulin core particles by centrifugal concentration drying and cooling

In this Example, insulin (pI=5.3) was used as a protein drug, and insulin core particles (uncoated) were prepared by removing a part of water from an insulin solution containing an ionic crystalline compound by centrifugal concentration to allow precipitation of insulin, and promoting precipitation by cooling.

The preparation included the following steps.

Hydrochloric acid (0.02 M) (3 ml) was added to dissolve Bovine Insulin (Sigma) (30 mg), and 2 ml of sodium citrate (0.2 M), 24 μl of zinc chloride solution (0.12 g/ml), and 0.36 g of sodium chloride were added and mixed therewith. Thereafter, a small amount of NaOH (0.1 M) was added to adjust the pH to 6.25. The obtained solution (1 ml) was transferred to a glass vial, and dried without heating by centrifugal concentrator under reduced pressure for 15 min to remove a part of water. Then, the solution was cooled at 4°C overnight to continue crystal precipitation, whereby an insulin particle suspension was obtained.

The size of the particles in the obtained suspension was measured. As a result, the diameter of the particles in the main resultant product was 390 nm (Fig. 4). Therefore, it was confirmed that insulin core particles having good and uniform particle size can be obtained by drying without heating by centrifugal concentrator under reduced pressure, even without complete drying.

Comparative Example 2: preparation of insulin core particles by centrifugal concentration drying of insulin solution free of ionic crystalline compound

In this Comparative Example, insulin (pI=5.3) was used as a protein drug, and insulin core particles (uncoated) were prepared by removing water from an insulin solution free of an ionic crystalline compound by centrifugal concentration drying to allow precipitation of insulin.

The preparation included the following steps.

Hydrochloric acid (0.02 M) (3 ml) was added to dissolve Bovine Insulin (Sigma) (30 mg), and 2 ml of additive solution (trisodium citrate dihydrate 58 mg/ml, zinc chloride 1.4 mg/ml) was added. Thereafter, NaOH (0.1 M) was added to adjust the pH to 6.25. The obtained solution (0.25 ml) was transferred to a glass vial, and dried without heating by centrifugal concentrator under reduced pressure to remove water. Water (2 ml) was added to the dried product to give a suspension for particle size measurement.

The size of the particles in the obtained suspension was measured. As a result, the diameter of the particles in the main resultant product was 3200 nm (Fig. 5). When the surface of core particles is coated, the size grows more than that. Therefore, a particle size of this level is not suitable for use desired in the present invention, particularly, use for vaccine, and is not preferable.

From the results of Examples 1 - 3 and Comparative Examples 1 - 2, it is clear that addition of an ionic crystalline compound to an insulin solution, and insulin precipitation by drying without heating under reduced pressure are effective for preparation of insulin core particles (uncoated) having an appropriate particle size. It is considered that insulin precipitation and preparation of ionic crystalline compound simultaneously proceed by drying without heating under reduced pressure, thereby preventing excessive aggregation and of large size of the precipitated insulin particles.

Example 4: preparation of surface-coated small particle by coating insulin core particle prepared by centrifugal concentration drying with chitosan

In this Example, surface-coated small particles were prepared by coating insulin core particles prepared by centrifugal concentration drying with chitosan.

The preparation included the following steps.

Hydrochloric acid (0.02 M) (3 ml) was added to dissolve Bovine Insulin (Sigma) (30 mg), and 2 ml of salt solution (trisodium citrate dihydrate 58 mg/ml, zinc chloride 1.4 mg/ml, sodium chloride 180 mg/ml) was added. Thereafter, NaOH (0.1 M) was added to adjust the pH to 6.25. The obtained solution (0.25 ml) was transferred to a glass vial, and dried without heating by centrifugal concentrator under reduced pressure to remove water. To the dried product was added 1 ml of chitosan solution (manufactured by Dainichiseika Co., Ltd., aqueous chitosan solution) prepared to 1% (w/v) to give a resuspension, which was placed in a dialysis tube (molecular weight cutoff value 10000) and dialyzed against 500 ml of 5 mM MES buffer (pH 6.0) for 60 min. A suspension after dialysis was obtained as a sample.

The size of the particles in the obtained suspension was measured. As a result, the diameter of the particles in the main resultant product was 1800 nm (Fig. 6). As compared to the insulin core particles of Example 1 prepared by a similar method, the particle size increased.

In addition, the zeta potential of the particles in the obtained suspension was measured. As a result, the zeta

potential was +19 mV at pH 6. The insulin core particles of Example 1 were charged minus reflecting the minus charge of insulin at pH 6. The small particles obtained in this Example had a plus charge derived from the plus charge of chitosan at pH 6.

Therefrom it is clear that insulin core particles were coated fine with chitosan.

Example 5: preparation of surface-coated small particles by coating insulin core particles prepared by freeze-drying with chitosan

In this Example, insulin core particles prepared by freeze-drying were coated with chitosan, whereby surface-coated small particles were prepared.

The preparation included the following steps.

Hydrochloric acid (0.02 M) (3 ml) was added to dissolve Bovine Insulin (Sigma) (30 mg), and 2 ml of salt solution (trisodium citrate dihydrate 58 mg/ml, zinc chloride 1.4 mg/ml, sodium chloride 180 mg/ml) was added. Thereafter, NaOH (0.1 M) was added to adjust the pH to 6.25. The obtained solution (0.25 ml) was transferred to a glass vial, frozen with liquid nitrogen, and dried without heating by freeze-drier under reduced pressure to remove water. To the dried product was added 1 ml of chitosan solution (manufactured by Dainichiseika Co., Ltd., aqueous chitosan solution) prepared to 1% (w/v) to give a resuspension, which was placed in a dialysis tube (molecular weight cutoff value 10000) and dialyzed against 500 ml of 5 mM MES buffer (pH 6.0) for 60 min. A suspension after dialysis was obtained as a sample.

The size of the particles in the obtained suspension was measured. As a result, the diameter of the particles in the main resultant product was 1500 nm (Fig. 7). As compared to the insulin core particles of Example 2 prepared by a similar method, the particle size increased.

In addition, the zeta potential of the particles in the obtained suspension was measured. As a result, the zeta potential was +17 mV at pH 6.

Therefrom it is clear that surface-coated small particles having a small particle size can also be obtained by a method using freeze-drying as in Example 4.

Example 6: preparation of surface-coated small particles by coating insulin core particles with DMAEMA-PEG (without dialysis)

In this Example, insulin core particles prepared by centrifugal concentration drying were coated with DMAEMA-PEG, whereby surface-coated small particles were prepared. For preparation, dialysis was not performed.

The preparation included the following steps.

Hydrochloric acid (0.02 M) (3 ml) was added to dissolve Bovine Insulin (Sigma) (30 mg), and 2 ml of trisodium citrate (0.2 M), 24 μl of zinc chloride solution (0.12 g/ml), and 0.36 g of sodium chloride were added. Thereafter, a small amount of NaOH (0.1 M) was added to adjust the pH to 6.25. The obtained solution (1 ml) was transferred to a glass vial, and dried without heating by centrifugal concentrator under reduced pressure to completely remove water. To the dried product was added 1 ml of 0.1% w/v aqueous DMAEMA-stat-PEGMA solution (pH 6.0) to give a resuspension as a sample.

DMAEMA-stat-PEGMA is a compound having the following chemical formula:

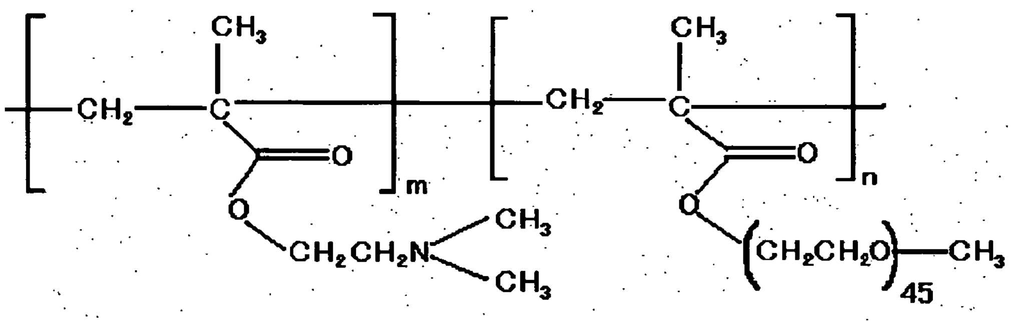

Mn=21,000, m=66, n=5, Mw/Mn=1.11

The size of the particles in the obtained suspension was measured. As a result, the diameter of the particles in the main resultant product was 260 nm (Fig. 8).

Example 7: preparation of surface-coated small particles by coating insulin core particles with DMAEMA-PEG (with dialysis)

A suspension prepared in the same manner as in Example 6 was placed in a dialysis tube (molecular weight cutoff value 10000) and dialyzed against 500 ml of 0.5 mM citric acid solution (pH 6.0) for 60 min. A suspension after dialysis was obtained as a sample.
The size of the particles in the obtained suspension was measured. As a result, the diameter of the particles in the main resultant product was 260 nm (Fig. 9).
In Examples 6 and 7, DMAEMA-PEG was used as a surface-coating polymer. In this case, it was confirmed that stable surface-coated small particles having a small particle size can be prepared irrespective of use of dialysis.

Example 8: preparation of surface-coated small particles by coating insulin core particles with PEG-modified chitosan

In this Example, insulin core particles prepared by centrifugal concentration drying were coated with PEG-modified chitosan, whereby surface-coated small particles were prepared.
The preparation included the following steps.
Hydrochloric acid (0.02 M) (3 ml) was added to dissolve Bovine Insulin (Sigma) (30 mg), and 2 ml of trisodium citrate (0.2 M), 24 μl of zinc chloride solution (0.12 g/ml), and 0.36 g of sodium chloride were added. Thereafter, a small amount of NaOH (0.1 M) was added to adjust the pH to 6.25. The obtained solution (1 ml) was transferred to a glass vial, and dried without heating by centrifugal concentrator under reduced pressure to completely remove water. To the dried product was added 1 ml of 0.1% w/v aqueous solution (pH 6.0) of PEG-modified chitosan (manufactured by Carbomer.Inc.) to give a resuspension as a sample.
The size of the particles in the obtained suspension was measured. As a result, the diameter of the particles in the main resultant product was 510 nm (Fig. 10).
It was confirmed that stable surface-coated small particles having a small particle size can also be prepared when PEG-modified chitosan was used as a surface-coating polymer.

Example 9: preparation of surface-coated small particles by coating egg albumin core particles with chitosan

In this Example, surface-coated small particles were prepared by coating egg albumin core particles prepared by centrifugal concentration drying with chitosan.
The preparation included the following steps.
Hydrochloric acid (0.02 M) (2.4 ml) was added to dissolve egg albumin (Sigma) (24 mg), and 1.6 ml of salt solution (trisodium citrate dihydrate 59 mg/ml, sodium chloride 180 mg/ml) was added. Thereafter, NaOH (0.1 M) was added to adjust the pH to 6.25. The obtained solution (0.25 ml) was transferred to a glass vial, and dried without heating by centrifugal concentrator under reduced pressure to remove water. To the dried product was added 1 ml of 1.5% (w/v) chitosan solution (manufactured by Dainichiseika Co., Ltd., aqueous chitosan solution) to give a resuspension, which was placed in a dialysis tube (molecular weight cutoff value 10000) and dialyzed against 500 ml of 5 mM MES buffer (pH 6.0) for 60 min. A suspension after dialysis was obtained as a sample.
The size of the particles in the obtained suspension was measured. As a result, the diameter of the particles in the main resultant product was 600 nm (Fig. 11).
In addition, the zeta potential of the particles in the obtained suspension was measured. As a result, the zeta potential was +12 mV at pH 6.
In this Example, coated particles were prepared using egg albumin as a protein drug and by centrifugal concentration drying excluding zinc chloride. It was confirmed that surface-coated small particles having a small particle size can be prepared in this case.

Example 10: preparation of surface-coated small particles by coating egg albumin core particles with cationic chitosan derivative (without dialysis)

In this Example, surface-coated small particles were prepared by coating egg albumin core particles prepared by freeze-drying with chitosan. For preparation, dialysis was not performed.
The preparation included the following steps.
Hydrochloric acid (0.02 M) (2.4 ml) was added to dissolve egg albumin (Sigma) (24 mg), and 1.6 ml of salt solution (trisodium citrate dihydrate 59 mg/ml, sodium chloride 180 mg/ml) was added. Thereafter, NaOH (0.1 M) was added to adjust the pH to 6.25. The obtained solution (0.25 ml) was transferred to a glass vial, frozen with liquid nitrogen, and dried without heating by freeze-drier under reduced pressure to remove water. To the dried product was resuspended in 1 ml of 1.1% (w/v) cationic aqueous chitosan derivative solution (manufactured by Dainichiseika Co., Ltd., cationic

chitosan derivative) to give a surface-coated small particles. Then, the particles were diluted with 5 mM MES buffer (pH 6.5) to give a sample having an egg albumin concentration of 62.5 μg/ml.

The size of the particles in the obtained suspension was measured. As a result, the diameter of the particles in the main resultant product was 260 nm (Fig. 12).

In addition, the zeta potential of the particles in the obtained suspension was measured. As a result, the zeta potential was +21 mV at pH 6.5.

Example 11: preparation of surface-coated small particles by coating egg albumin core particles with cationic chitosan derivative (with dialysis)

In this Example, egg albumin core particles prepared by freeze-drying were coated with chitosan to give surface-coated small particles. For preparation, dialysis was performed.

The preparation included the following steps.

Hydrochloric acid (0.02 M) (2.4 ml) was added to dissolve egg albumin (Sigma) (24 mg), and 1.6 ml of salt solution (trisodium citrate dihydrate 59 mg/ml, sodium chloride 180 mg/ml) was added. Thereafter, NaOH (0.1 M) was added to adjust the pH to 6.25. The obtained solution (0.25 ml) was transferred to a glass vial, frozen with liquid nitrogen, and dried without heating by freeze-drier under reduced pressure to remove water. To the dried product was added 1 ml of 1.1% (w/v) cationic chitosan solution (manufactured by Dainichiseika Co., Ltd., cationic chitosan derivative) to give a resuspension, which was placed in a dialysis tube (molecular weight cutoff value 10000) and dialyzed twice each against 500 ml of 5 mM MES buffer (pH 6.5) for 30 min. A suspension after dialysis was obtained as a sample.

The size of the particles in the obtained suspension was measured. As a result, the diameter of the particles in the main resultant product was 484 nm (Fig. 13).

In addition, the zeta potential of the particles in the obtained suspension was measured. As a result, the zeta potential was +21 mV at pH 6.5.

In Examples 10 and 11, egg albumin was used as a protein drug and a cationic chitosan derivative was used as a surface-coating polymer. In these cases, it was confirmed that stable surface-coated small particles having a small particle size can be prepared irrespective of use of dialysis.

Comparative Example 3: preparation of small particles using Tween 80 instead of polymer

In this Comparative Example, preparation of surface-coated small particles was tried in the same manner as in Example 6 except that Tween 80 was used instead of aqueous DMAEMA-stat-PEGMA solution.

The preparation included the following steps.

Hydrochloric acid (0.02 M) (3 ml) was added to dissolve Bovine Insulin (Sigma) (30 mg), and 2 ml of trisodium citrate (0.2 M), 24 μl of zinc chloride solution (0.12 g/ml), and 0.36 g of sodium chloride were added. Thereafter, a small amount of NaOH (0.1 M) was added to adjust the pH to 6.25. The obtained solution (1 ml) was transferred to a glass vial, and dried without heating by centrifugal concentrator under reduced pressure to completely remove water. To the dried product was added 1 ml of 0.1% w/v aqueous solution of Tween 80 (pH 6.0) to give a resuspension as a sample.

The particle size of the particles in the obtained suspension was measured. As a result, the diameter of the particles in the main resultant product was over 5000 nm (Fig. 14).

Comparative Example 4: preparation of small particles at pH different from Example 8

In this Example, small particles were prepared in the same manner as in Example 8 except that pH was set to 4.0 instead of 6.5.

The preparation included the following steps.

Hydrochloric acid (0.02 M) (3 ml) was added to dissolve Bovine Insulin (Sigma) (30 mg), and 2 ml of trisodium citrate (0.2 M), 24 μl of zinc chloride solution (0.12 g/ml), and 0.36 g of sodium chloride were added. Thereafter, a small amount of HCl (0.1 M) was added to adjust the pH to 4.0. The obtained solution (1 ml) was transferred to a glass vial, and dried without heating by centrifugal concentrator under reduced pressure to completely remove water. To the dried product was added 1 ml of 0.1% w/v aqueous solution (pH 4.0) of PEG-modified chitosan (manufactured by Carbomer. Inc.) to give a resuspension as a sample.

The size of the particles in the obtained suspension was measured. As a result, the diameter of the particles in the main resultant product was above 5000 nm (Fig. 15).

Experimental Example 1: electron microscope observation and elemental analysis of small particles

The samples of Examples 10 and 11 were subjected to observation by FE-TEM (HITACHI, HF-2000, acceleration voltage 200 kV) by dispersion on Cu mesh with a carbon support film and elemental analysis of the inside of small particles by XMA (Kevex, Sigma, energy dispersion type).

Fig. 16 shows a TEM image of small particles of Example 10. In the small particles of Example 10, surface-coating polymer could not be observed since the electron density was low. However, egg albumin particles per se could be observed. Square crystals were observed in the inside of the particles. As a result of the elemental analysis of the crystal part by XMA, it was found to be a crystal of sodium chloride since Na and Cl were contained in large amounts (Fig. 17). Also, in the TEM image (Fig. 18) of the small particles of Example 11 in which sodium chloride attached to the surface of small particles had been removed by dialysis, square crystals were observed in the inside of the particles and, as a result of the elemental analysis of the crystal part by XMA, it was found to be a crystal of sodium chloride since Na and Cl were contained in large amounts (Fig. 19).

Experimental Example 2: analysis of insulin encapsulation efficiency in surface-coated small particles

Supernatant (free insulin) was removed from chitosan-coated insulin particles prepared by the method of Example 4 by centrifugation (10,000×G, 30 min) to give a particle fraction. 33% Acetic acid solution was added to the particle fraction to extract insulin and the insulin concentration was quantified by HPLC. The encapsulation efficiency was calculated by comparison with the amount of insulin added by the following calculation formulas:

encapsulation efficiency (%) of added insulin
=100 x (insulin content mg/ml of prepared surface-coated small particle suspension)
x (total amount ml of prepared surface-coated small particle suspension)/(total amount mg of added insulin).

HPLC analysis of the samples was performed under the following conditions:

C18 column (Inertsil ODS-2, 5 μm, 250 mm × 4.6 mm);
mobile phase A: 0.1% aqueous TFA solution, mobile phase B: 0.1% TFA $CH_3CN$ solution;
gradient conditions (mobile phase B concentration): at 0 min: 30%, at 10 min: 50%;
column oven temperature: 40°C, flow rate: 1.0 ml/min, injection volume: 50 μl, detection: UV 275 nm.

As a result, the encapsulation efficiency of the added insulin was calculated to be 88%. Therefrom it was confirmed that the insulin encapsulation efficiency by the above-mentioned preparation method including dialysis operation was as high as 88%, and insulin was found to be encapsulated in the particles without a high degree of loss.

Experimental Example 3: observation of changes in appearance by preservation at room temperature

The samples obtained in Example 1 and Example 4 were diluted with 5 mM MES buffer and visually observed for the initial appearance and the appearance after standing at room temperature for one day.

The results are shown in the following Table 1.

Table 1 observation of changes in appearance

| | initial | after preservation for one day at room temperature |
|---|---|---|
| Example 1 | Colloid suspension | completely dissolved |
| Example 4 | Colloid suspension | Colloid suspension |

Small particles not coated with a polymer show poor stability as colloid particles, whereas surface-coated small particles were stable without dissolution in water even after long-term preservation. Therefore, it is clear that coating with polymer can delay dissolution of insulin. From the above results, use of the surface-coated small particles of the present invention suggests the possibility of conferring sustained-releaseability to a natural polymer in vaccine use or DDS use.

Experimental Example 4: stability against enzyme test of chitosan-coated insulin small particles

0.5 ml of the chitosan-coated insulin small particle suspension (insulin 160 μg/ml, 5 mM MES buffer (pH 6)) of Example 4 or insulin solution (insulin 160 μg/ml, 5 mM MES buffer (pH 6)) was mixed with 0.5 ml of model enzyme solution (α-chymotrypsin (Sigma Ltd.) dissolved in 5 mM MES buffer (pH 6) at a concentration of 40 μg/ml) and subjected to shaking reaction at 37°C for 30 min. After the reaction, cooled acetic acid (0.5 ml) was added and mixed therewith to quench the enzyme reaction as well as simultaneously dissolve insulin, and insulin concentration was quantified by HPLC, based on which stability of insulin in the chitosan-coated insulin small particles and stability of insulin in the insulin solution were evaluated. As a control experiment, an experiment including adding 5 mM MES buffer (pH 6) instead of the model enzyme solution, and performing a shaking reaction at 37°C for 30 min, followed by quantification of insulin concentration in the same manner as above, was also performed.

HPLC analysis of the samples was performed under the following conditions:

C18 column (Inertsil ODS-2, 5 μm, 250 mm × 4.6 mm);
mobile phase A: 0.1% aqueous TFA solution, mobile phase B: 0.1% TFA $CH_3CN$ solution;
gradient conditions (mobile phase B concentration): at 0 min: 30%, at 10 min: 50%;
column oven temperature: 40°C, flow rate: 1.0 ml/min, injection volume: 50 μl, detection: UV 275 nm.

The results are shown in Fig. 20. The sample of a mixture of insulin and enzyme showed a decrease by almost 50% in the insulin content after shaking reaction for 30 min, whereas the sample of a mixture of surface-coated small particles and enzyme, the sample of a mixture of surface-coated small particles and buffer, and the sample of a mixture of insulin solution and buffer showed almost equivalent insulin contents even after shaking reaction for 30 min.

From these results, it was confirmed that an insulin protection effect against enzyme decomposition can be obtained by coating insulin core particles with a polymer.

Experimental Example 5: immunity experiment of small particles of Example 10

200 μL of small particles of Example 10 (egg albumin concentration 62.5 μg/ml, pH 6.5) or egg albumin solution (egg albumin concentration 62.5 μg/ml, pH 6.5) was intradermally injected to the back of mouse (C57BL/6 lineage). Injection at one week interval was repeated twice, totaling 3 injections. One week after final injection, the humoral immunity level and cellular immunity level of the mouse were evaluated. The humoral immunity level was evaluated by measuring the amount of antibody against OVA in the serum based on the absorbance by ELISA method. The cellular immunity level was evaluated by stimulating the isolated spleen cells with OVA peptide, counting the spot number of the ELISPOT method, and measuring the secreted amount of IFN-γ.

The results are shown in the following Table 2.

Table 2 results of mouse immunity experiment of small particles of Example 10 (N=2)

| | humoral immunity level of serum (absorbance at 450 nm by ELISA analysis) | cellular immunity level of spleen cell (SPOT number of ELISPOT analysis) |
|---|---|---|
| Example 10 | 0.56, 0.65 (average 0.61) | 160, 400 (average 280) |
| OVA solution | 0.23, 0.40 (average 0.32) | 77, 46 (average 62) |

The small particles of Example 10 were administered to mouse. As a result, both humoral immunity and cellular immunity were confirmed to have been strongly induced as compared to administration of the control solution. Such results indicate that the small particles of the present invention are useful for vaccine use.

[Industrial Applicability]

Using the pharmaceutical composition of the present invention, efficient transmucosal or transdermal administration of a water-soluble drug has been enabled. Such pharmaceutical composition can be produced by the production method of the present invention.

The present invention is based on a patent application No. 2009-231001 filed on October 2, 2009 in Japan.

## Claims

1. A pharmaceutical composition comprising

   a small particle comprised of (a) a water-soluble drug and (b) a pharmaceutically acceptable ionic crystalline compound which is solid at room temperature, and
   a pharmaceutically acceptable polymer,

   wherein the ionic crystalline compound is crystallized in the small particle; and the small particle has a positive or negative charge at a predetermined pH, and the polymer has a charge of a sign opposite to that of the small particle at said pH, whereby the small particle and the polymer electrostatically interact with each other at said pH to form a complex wherein the polymer is attached to the surface of the small particle.

2. The pharmaceutical composition of claim 1, wherein the small particle is free of irreversible crosslinking.

3. The pharmaceutical composition of claim 1 or 2, wherein the small particle has an average particle size of not less than 10 nm and not more than 5000 nm.

4. The pharmaceutical composition of any one of claims 1 to 3, wherein the water-soluble drug is selected from the group consisting of peptide, protein, DNA, RNA, siRNA, polysaccharide, lipopeptide, lipoprotein, lipopolysaccharide, low-molecular-weight compound, antibody, antigen, toxin and vaccine.

5. The pharmaceutical composition of any one of claims 1 to 4, wherein the ionic crystalline compound comprises as constituent components, one or more types of cationic electrolytes selected from the group consisting of sodium ion, potassium ion, calcium ion, magnesium ion, zinc ion, iron ion and ammonium ion, and one or more types of anionic electrolytes selected from the group consisting of chlorine ion, sulfuric acid ion, lactic acid ion, acetic acid ion, phosphoric acid ion, gluconic acid ion, carbonate ion and bicarbonate ion.

6. The pharmaceutical composition of any one of claims 1 to 5, wherein the water-soluble drug has a weight ratio of not less than 0.001 and not more than 100 relative to the ionic crystalline compound.

7. The pharmaceutical composition of any one of claims 1 to 6, wherein the complex has an average particle size of not less than 15 nm and not more than 6000 nm.

8. A method of producing the pharmaceutical composition of any one of claims 1 to 7, comprising drying without heating an aqueous solution of the water-soluble drug and the ionic crystalline compound under reduced pressure to give the small particle and mixing the solution of a polymer with said pH and the small particle.

9. The method of claim 8, wherein the drying is freeze-drying or drying by centrifugal concentration.

10. A production method of a pharmaceutical composition comprising a small particle comprised of (a) a water-soluble drug and (b) a pharmaceutically acceptable ionic crystalline compound which is solid at room temperature, the ionic crystalline compound being crystallized in the small particle, which method comprises drying without heating an aqueous solution of the water-soluble drug and the ionic crystalline compound under reduced pressure, optionally wherein the small particle is free of irreversible crosslinking.

11. The method of claim 10, wherein the small particle has an average particle size of not less than 10 nm and not more than 5000 nm.

12. The method of claim 10 or claim 11, wherein the water-soluble drug is selected from the group consisting of peptide, protein, DNA, RNA, siRNA, polysaccharide, lipopeptide, lipoprotein, lipopolysaccharide, low-molecular-weight compound, antibody, antigen, toxin and vaccine.

13. The method of any one of claims 10 to 12, wherein the ionic crystalline compound comprises as constituent components, one or more types of cationic electrolytes selected from the group consisting of sodium ion, potassium ion, calcium ion, magnesium ion, zinc ion, iron ion and ammonium ion, and one or more types of anionic electrolytes selected from the group consisting of chlorine ion, sulfuric acid ion, lactic acid ion, acetic acid ion, phosphoric acid ion, gluconic acid ion, carbonate ion and bicarbonate ion.

14. The method of any one of claims 10 to 13, wherein the water-soluble drug has a weight ratio of not less than 0.001 and not more than 100 relative to the ionic crystalline compound.

15. The method of any one of claims 10 to 14, wherein the drying is freeze-drying or drying by centrifugal concentration.

## Patentansprüche

1. Pharmazeutische Zusammensetzung, umfassend:

   ein kleines Teilchen, das aus (a) einem wasserlöslichen Wirkstoff und (b) einer pharmazeutisch annehmbaren ionischen kristallinen Verbindung, die bei Raumtemperatur fest ist, besteht; und
   ein pharmazeutisch annehmbares Polymer;
   wobei die ionische kristalline Verbindung in dem kleinen Teilchen kristallisiert ist und das kleine Teilchen bei einem vorbestimmten pH-Wert eine positive oder negative Ladung aufweist und das Polymer bei diesem pH-Wert eine Ladung mit einem zu der des kleinen Teilchens entgegengesetzten Vorzeichen aufweist, wodurch das kleine Teilchen und das Polymer bei diesem pH-Wert unter Bildung eines Komplexes elektrostatisch miteinander wechselwirken, wobei das Polymer an die Oberfläche des kleinen Teilchens gebunden ist.

2. Pharmazeutische Zusammensetzung gemäß Anspruch 1, wobei das kleine Teilchen frei von irreversibler Vernetzung ist.

3. Pharmazeutische Zusammensetzung gemäß Anspruch 1 oder 2, wobei das kleine Teilchen eine mittlere Teilchengröße von nicht weniger als 10 nm und nicht mehr als 5000 nm aufweist.

4. Pharmazeutische Zusammensetzung gemäß einem der Ansprüche 1 bis 3, wobei der wasserlösliche Wirkstoff aus der Gruppe ausgewählt ist, die aus einem Peptid, Protein, einer DNA, RNA, siRNA, einem Polysaccharid, Lipopeptid, Lipoprotein, Lipopolysaccharid, einer niedermolekularen Verbindung, einem Antikörper, Antigen, Toxin und Impfstoff besteht.

5. Pharmazeutische Zusammensetzung gemäß einem der Ansprüche 1 bis 4, wobei die ionische kristalline Verbindung als Bestandteile einen oder mehrere Typen kationischer Elektrolyten, die aus der Gruppe ausgewählt sind, die aus Natriumionen, Kaliumionen, Calciumionen, Magnesiumionen, Zinkionen, Eisenionen und Ammoniumionen besteht, und einen oder mehrere Typen anionischer Elektrolyten, die aus der Gruppe ausgewählt sind, die aus Chlorionen, Schwefelsäureionen, Milchsäureionen, Essigsäureionen, Phosphorsäureionen, Gluconsäureionen, Carbonationen und Hydrogencarbonationen besteht, umfasst.

6. Pharmazeutische Zusammensetzung gemäß einem der Ansprüche 1 bis 5, wobei der wasserlösliche Wirkstoff einen Gewichtsanteil von nicht weniger als 0,001 und nicht mehr als 100 relativ zu der ionischen kristallinen Verbindung aufweist.

7. Pharmazeutische Zusammensetzung gemäß einem der Ansprüche 1 bis 6, wobei der Komplex eine mittlere Teilchengröße von nicht weniger als 15 nm und nicht mehr als 6000 nm aufweist.

8. Verfahren zur Herstellung der pharmazeutischen Zusammensetzung gemäß einem der Ansprüche 1 bis 7, umfassend das Trocknen einer wässrigen Lösung des wasserlöslichen Wirkstoffs und der ionischen kristallinen Verbindung ohne Erhitzen und unter reduziertem Druck, was kleine Teilchen ergibt, und das Mischen der Lösung eines Polymers, die den pH-Wert aufweist, mit den kleinen Teilchen.

9. Verfahren gemäß Anspruch 8, wobei es sich bei dem Trocknen um Gefriertrocknen oder Trocknen durch zentrifugale Konzentration handelt.

10. Herstellungsverfahren für eine pharmazeutische Zusammensetzung, die ein kleines Teilchen, das aus (a) einem wasserlöslichen Wirkstoff und (b) einer pharmazeutisch annehmbaren ionischen kristallinen Verbindung, die bei Raumtemperatur fest ist, besteht, umfasst, wobei die ionische kristalline Verbindung in dem kleinen Teilchen kristallisiert ist, wobei das Verfahren das Trocknen einer wässrigen Lösung des wasserlöslichen Wirkstoffs und der ionischen kristallinen Verbindung ohne Erhitzen und unter reduziertem Druck umfasst, wobei das kleine Teilchen gegebenenfalls frei von irreversibler Vernetzung ist.

**11.** Verfahren gemäß Anspruch 10, wobei das kleine Teilchen eine mittlere Teilchengröße von nicht weniger als 10 nm und nicht mehr als 5000 nm aufweist.

**12.** Verfahren gemäß Anspruch 10 oder 11, wobei der wasserlösliche Wirkstoff aus der Gruppe ausgewählt ist, die aus einem Peptid, Protein, einer DNA, RNA, siRNA, einem Polysaccharid, Lipopeptid, Lipoprotein, Lipopolysaccharid, einer niedermolekularen Verbindung, einem Antikörper, Antigen, Toxin und Impfstoff besteht.

**13.** Verfahren gemäß einem der Ansprüche 10 bis 12, wobei die ionische kristalline Verbindung als Bestandteile einen oder mehrere Typen kationischer Elektrolyten, die aus der Gruppe ausgewählt sind, die aus Natriumionen, Kaliumionen, Calciumionen, Magnesiumionen, Zinkionen, Eisenionen und Ammoniumionen besteht, und einen oder mehrere Typen anionischer Elektrolyten, die aus der Gruppe ausgewählt sind, die aus Chlorionen, Schwefelsäureionen, Milchsäureionen, Essigsäureionen, Phosphorsäureionen, Gluconsäureionen, Carbonationen und Hydrogencarbonationen besteht, umfasst.

**14.** Verfahren gemäß einem der Ansprüche 10 bis 13, wobei der wasserlösliche Wirkstoff einen Gewichtsanteil von nicht weniger als 0,001 und nicht mehr als 100 relativ zu der ionischen kristallinen Verbindung aufweist.

**15.** Verfahren gemäß einem der Ansprüche 10 bis 14, wobei es sich bei dem Trocknen um Gefriertrocknen oder Trocknen durch zentrifugale Konzentration handelt.

## Revendications

**1.** Composition pharmaceutique comprenant
une petite particule constituée de (a) un médicament soluble dans l'eau et (b) un composé cristallin ionique pharmaceutiquement acceptable qui est solide à température ambiante, et
un polymère pharmaceutiquement acceptable,
dans laquelle le composé cristallin ionique est cristallisé dans la petite particule ; et la petite particule a une charge positive ou négative à un pH prédéterminé, et le polymère a une charge d'un signe opposé à celui de la petite particule audit pH, moyennant quoi la petite particule et le polymère interagissent électrostatiquement l'un avec l'autre audit pH pour former un complexe dans lequel le polymère est fixé à la surface de la petite particule.

**2.** Composition pharmaceutique selon la revendication 1, dans laquelle la petite particule est exempte de réticulation irréversible.

**3.** Composition pharmaceutique selon la revendication 1 ou 2, dans laquelle la petite particule a une granulométrie moyenne non inférieure à 10 nm et non supérieure à 5 000 nm.

**4.** Composition pharmaceutique selon l'une quelconque des revendications 1 à 3, dans laquelle le médicament soluble dans l'eau est choisi dans le groupe constitué de peptide, protéine, ADN, ARN, ARNsi, polysaccharide, lipopeptide, lipoprotéine, lipopolysaccharide, composé de faible poids moléculaire, anticorps, antigène, toxine et vaccin.

**5.** Composition pharmaceutique selon l'une quelconque des revendications 1 à 4, dans laquelle le composé cristallin ionique comprend en tant que composants constitutifs, un ou plusieurs types d'électrolytes cationiques choisis dans le groupe constitué de l'ion sodium, l'ion potassium, l'ion calcium, l'ion magnésium, l'ion zinc, l'ion fer et l'ion ammonium, et un ou plusieurs types d'électrolytes anioniques choisis dans le groupe constitué de l'ion chlore, l'ion acide sulfurique, l'ion acide lactique, l'ion acide acétique, l'ion acide phosphorique, l'ion acide gluconique, l'ion carbonate et l'ion bicarbonate.

**6.** Composition pharmaceutique selon l'une quelconque des revendications 1 à 5, dans laquelle le médicament soluble dans l'eau a un rapport pondéral non inférieur à 0,001 et non supérieur à 100 par rapport au composé cristallin ionique.

**7.** Composition pharmaceutique selon l'une quelconque des revendications 1 à 6, dans laquelle le complexe a une granulométrie moyenne non inférieure à 15 nm et non supérieure à 6000 nm.

**8.** Procédé de production de la composition pharmaceutique selon l'une quelconque des revendications 1 à 7, comprenant le séchage sans chauffage d'une solution aqueuse du médicament soluble dans l'eau et du composé cristallin ionique sous pression réduite pour donner la petite particule et le mélange de la solution d'un polymère

ayant ledit pH et de la petite particule.

9. Procédé selon la revendication 8, dans lequel le séchage est une lyophilisation ou un séchage par concentration centrifuge.

10. Procédé de production d'une composition pharmaceutique comprenant une petite particule constituée de (a) un médicament soluble dans l'eau et (b) un composé cristallin ionique pharmaceutiquement acceptable qui est solide à température ambiante, le composé cristallin ionique étant cristallisé dans la petite particule, lequel procédé comprend le séchage sans chauffage d'une solution aqueuse du médicament soluble dans l'eau et du composé cristallin ionique sous pression réduite, optionnellement dans lequel la petite particule est exempte de réticulation irréversible.

11. Procédé selon la revendication 10, dans lequel la petite particule a une granulométrie moyenne non inférieure à 10 nm et non supérieure à 5 000 nm.

12. Procédé selon la revendication 10 ou la revendication 11, dans lequel le médicament soluble dans l'eau est choisi dans le groupe constitué de peptide, protéine, ADN, ARN, ARNsi, polysaccharide, lipopeptide, lipoprotéine, lipopolysaccharide, composé de faible poids moléculaire, anticorps, antigène, toxine et vaccin.

13. Procédé selon l'une quelconque des revendications 10 à 12, dans lequel le composé cristallin ionique comprend en tant que composants constitutifs, un ou plusieurs types d'électrolytes cationiques choisis dans le groupe constitué de l'ion sodium, l'ion potassium, l'ion calcium, l'ion magnésium, l'ion zinc, l'ion fer et l'ion ammonium, et un ou plusieurs types d'électrolytes anioniques choisis dans le groupe constitué de l'ion chlore, l'ion acide sulfurique, l'ion acide lactique, l'ion acide acétique, l'ion acide phosphorique, l'ion acide gluconique, l'ion carbonate et l'ion bicarbonate.

14. Procédé selon l'une quelconque des revendications 10 à 13, dans lequel le médicament soluble dans l'eau a un rapport pondéral non inférieur à 0,001 et non supérieur à 100 par rapport au composé cristallin ionique.

15. Procédé selon l'une quelconque des revendications 10 à 14, dans lequel le séchage est une lyophilisation ou un séchage par concentration centrifuge.

FIG. 1

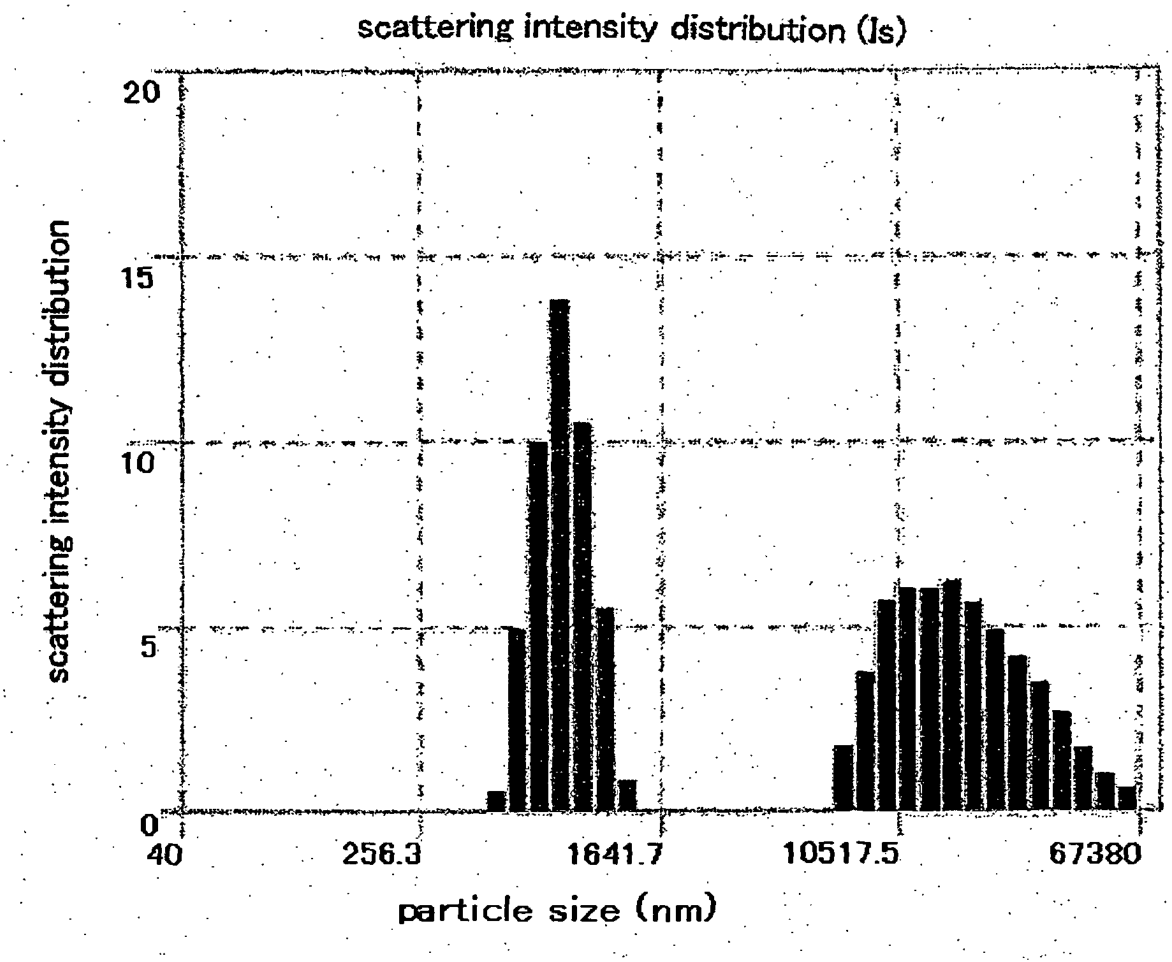
scattering intensity distribution (Js)
scattering intensity distribution
20
15
10
5
0
40
256.3
1641.7
10517.5
67380
particle size (nm)

FIG. 2

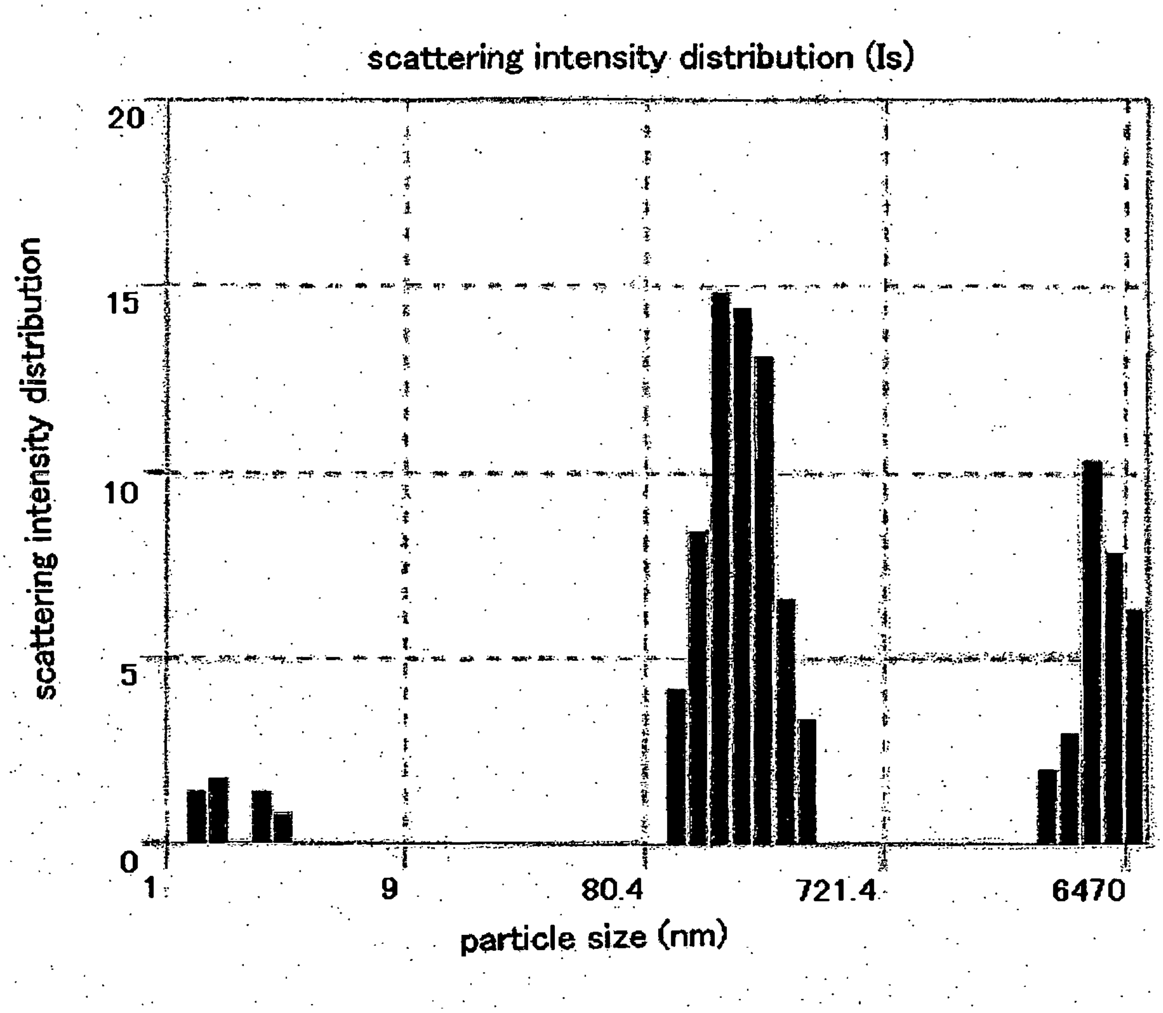
scattering intensity distribution (Is)
scattering intensity distribution
20
15
10
5
0
1
9
80.4
721.4
6470
particle size (nm)

FIG. 3

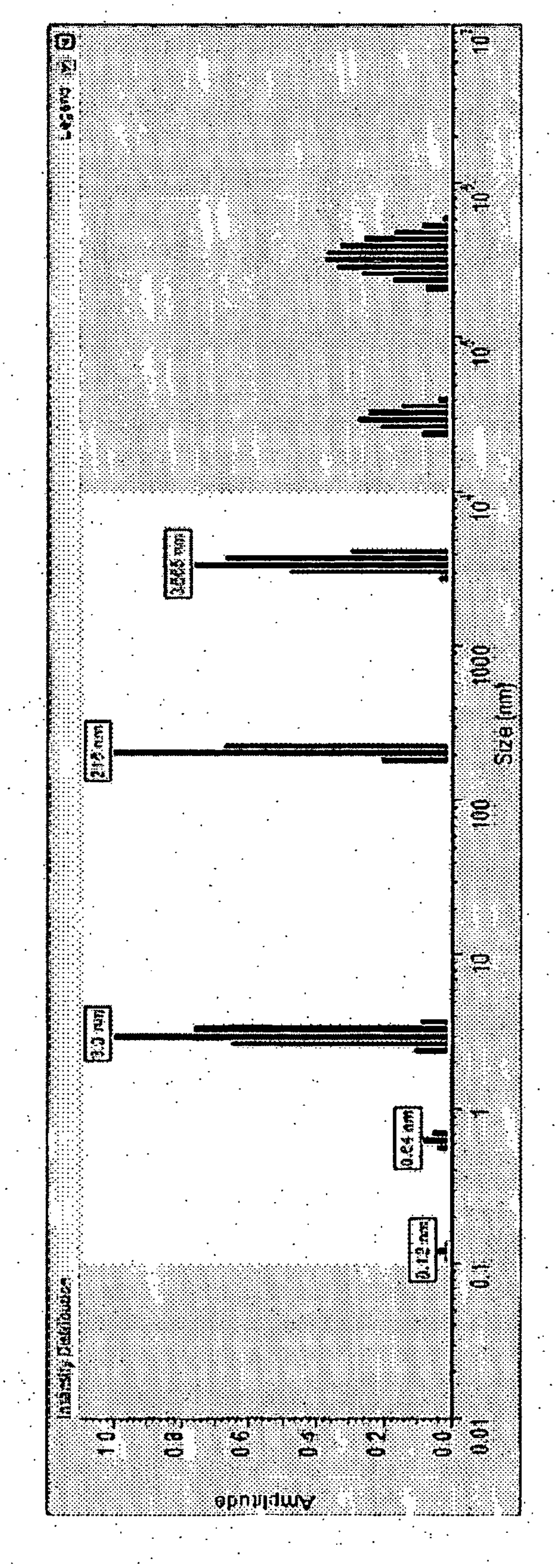
Intensity Distribution
Legend
Amplitude
Size (nm)
0.0
0.2
0.4
0.6
0.8
1.0
0.01
0.1
1
10
100
1000

FIG. 4

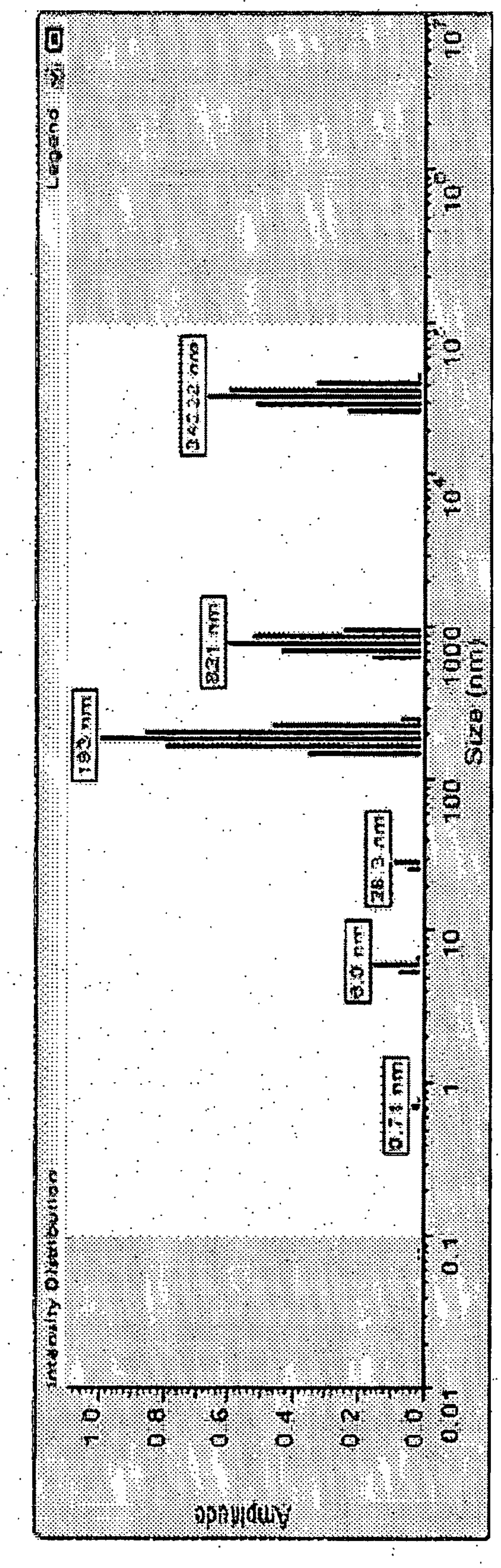
Intensity Distribution
Legend
Amplitude
Size (nm)
0.71 nm
6.0 nm
28.3 nm
193 nm
821 nm
0.0
0.2
0.4
0.6
0.8
1.0
0.01
0.1
1
10
100
1000

FIG. 5

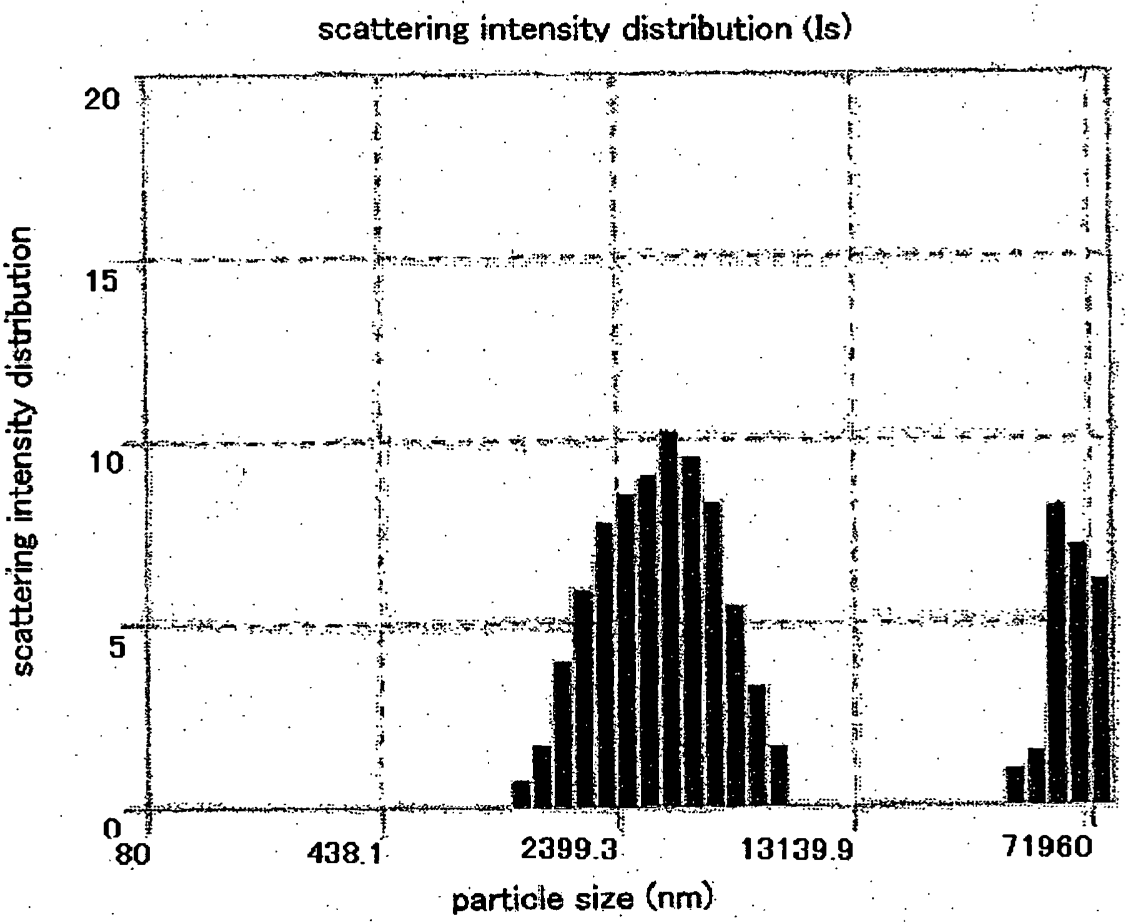
scattering intensity distribution (Is)
scattering intensity distribution
20
15
10
5
0
80
438.1
2399.3
13139.9
71960
particle size (nm)

FIG. 6

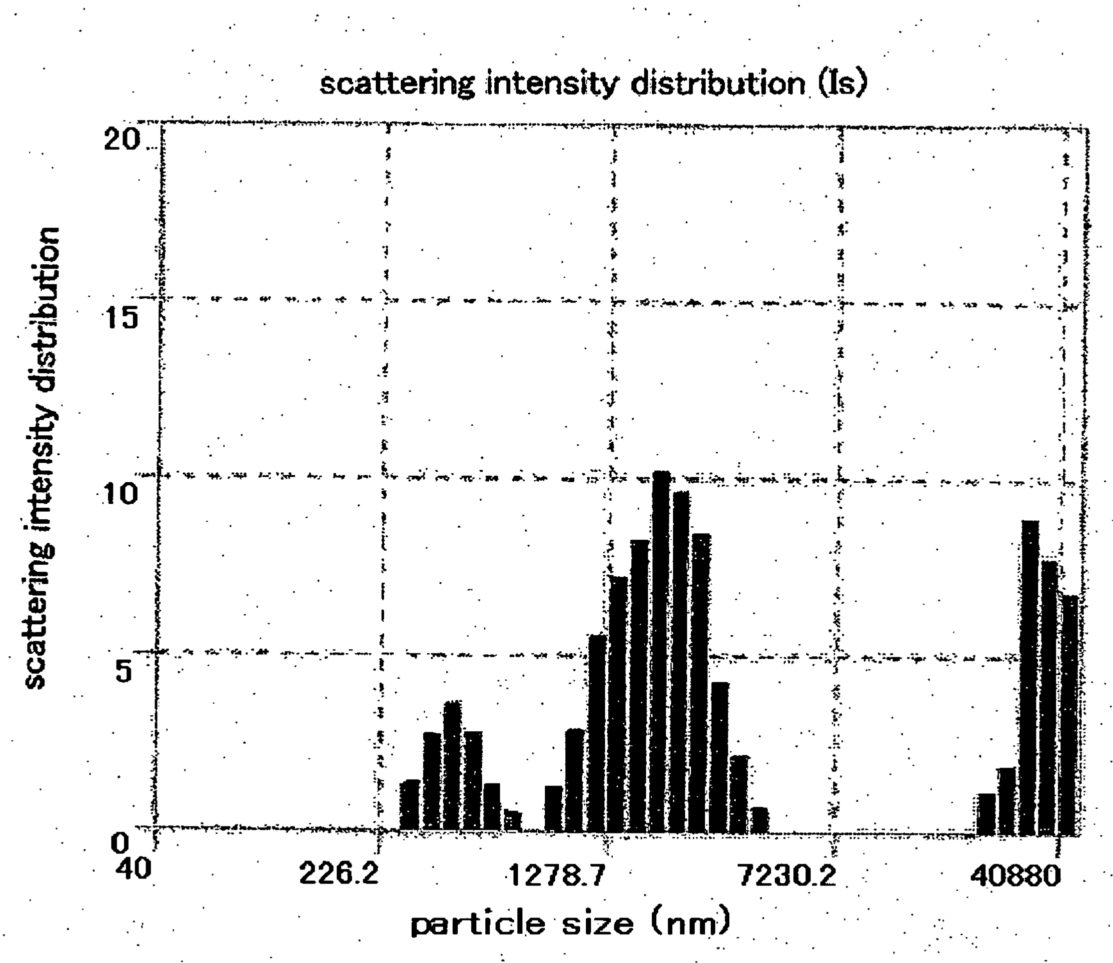
scattering intensity distribution (Is)
scattering intensity distribution
20
15
10
5
0
40
226.2
1278.7
7230.2
40880
particle size (nm)

FIG. 7

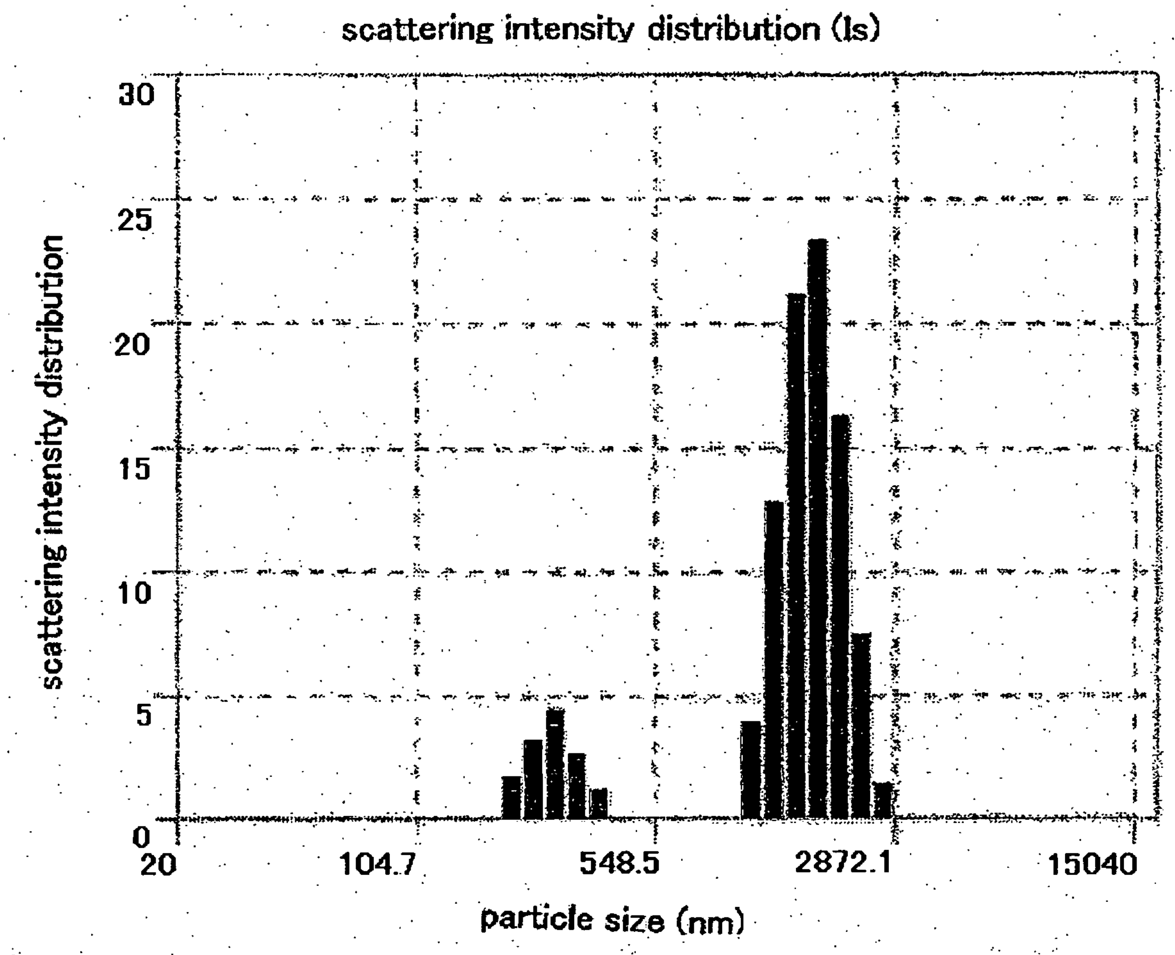
scattering intensity distribution (Is)
scattering intensity distribution
30
25
20
15
10
5
0
20
104.7
548.5
2872.1
15040
particle size (nm)

# FIG. 8

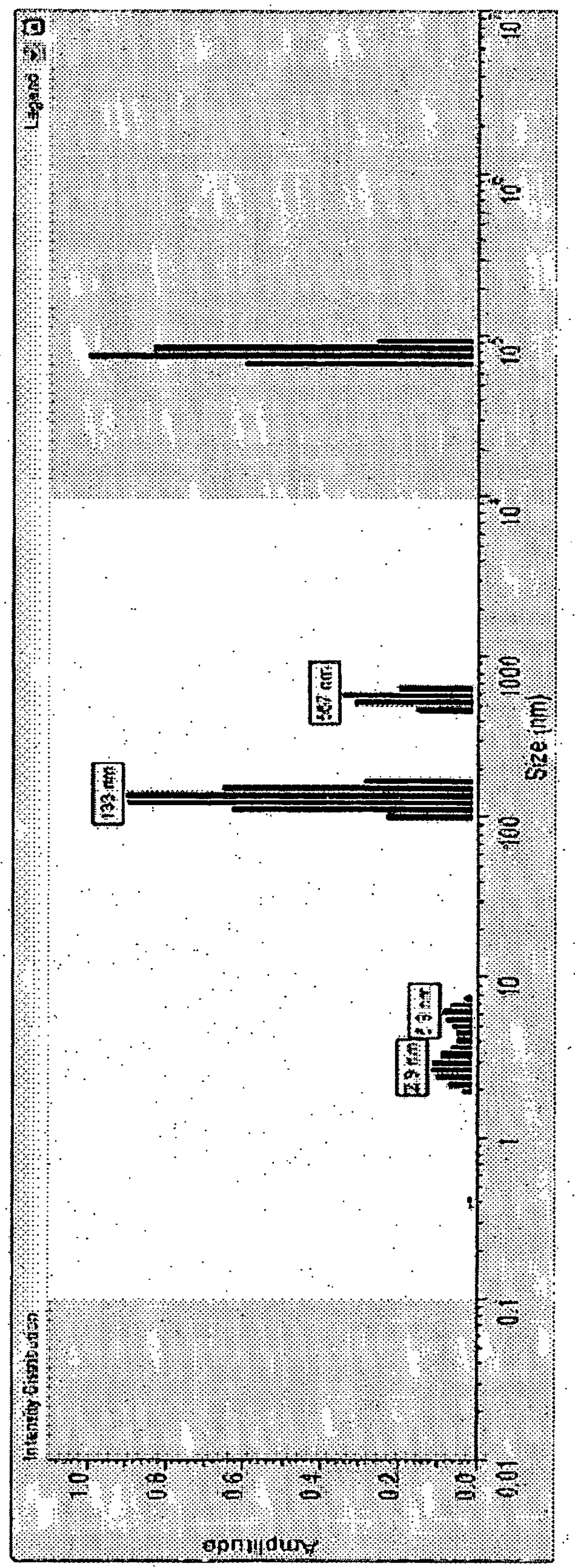
Intensity Distribution
Legend
133 nm
567 nm
Size (nm)
Amplitude

FIG. 9

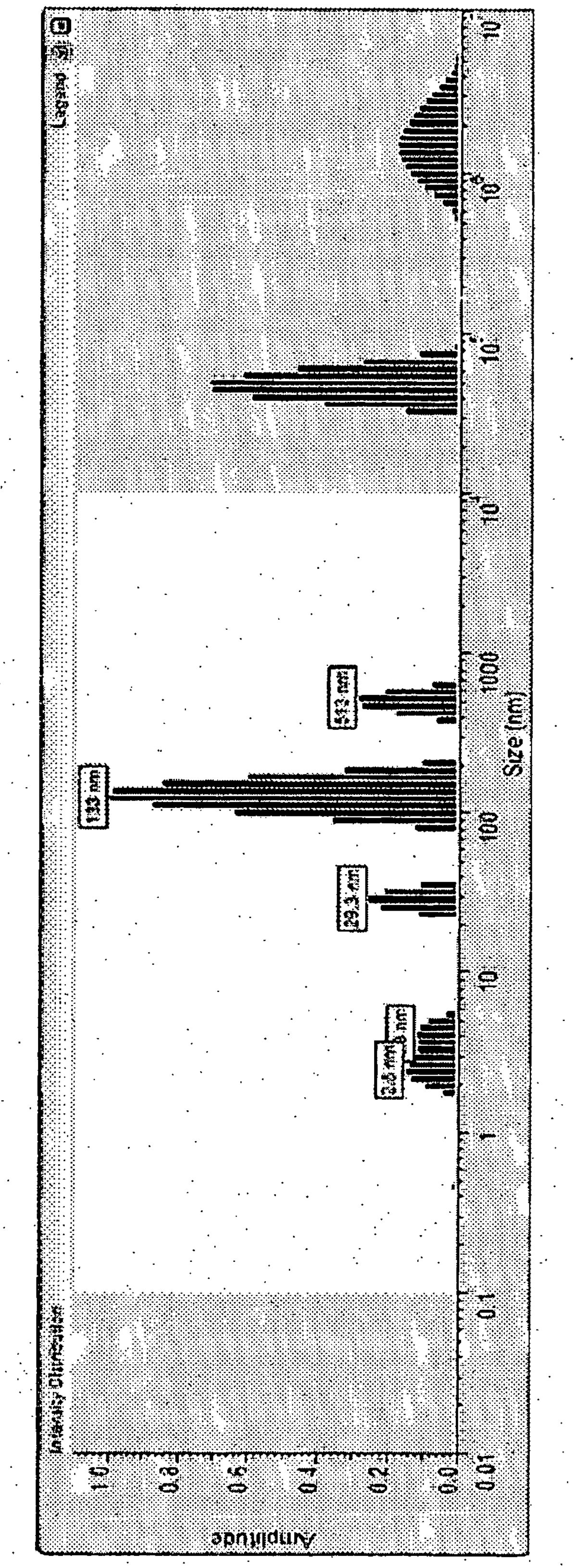
Legend
Amplitude
1.0
0.8
0.6
0.4
0.2
0.0
0.01
0.1
1
10
100
1000
Size (nm)
29.3 nm
133 nm
513 nm

FIG. 10

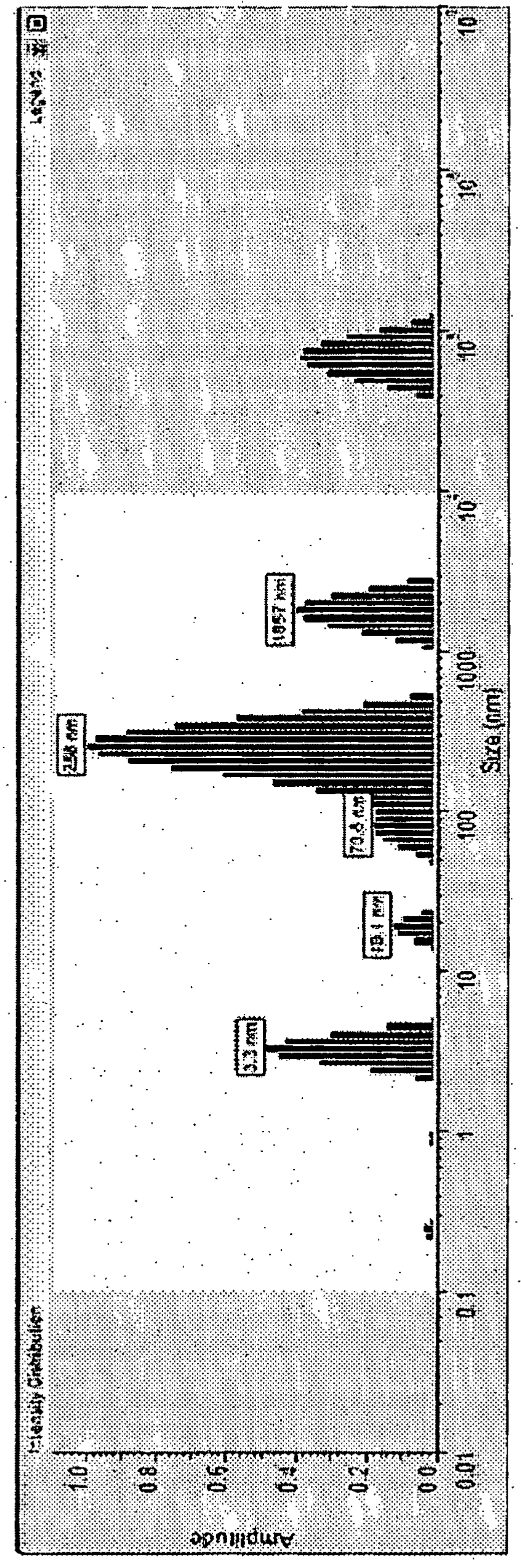

FIG. 11

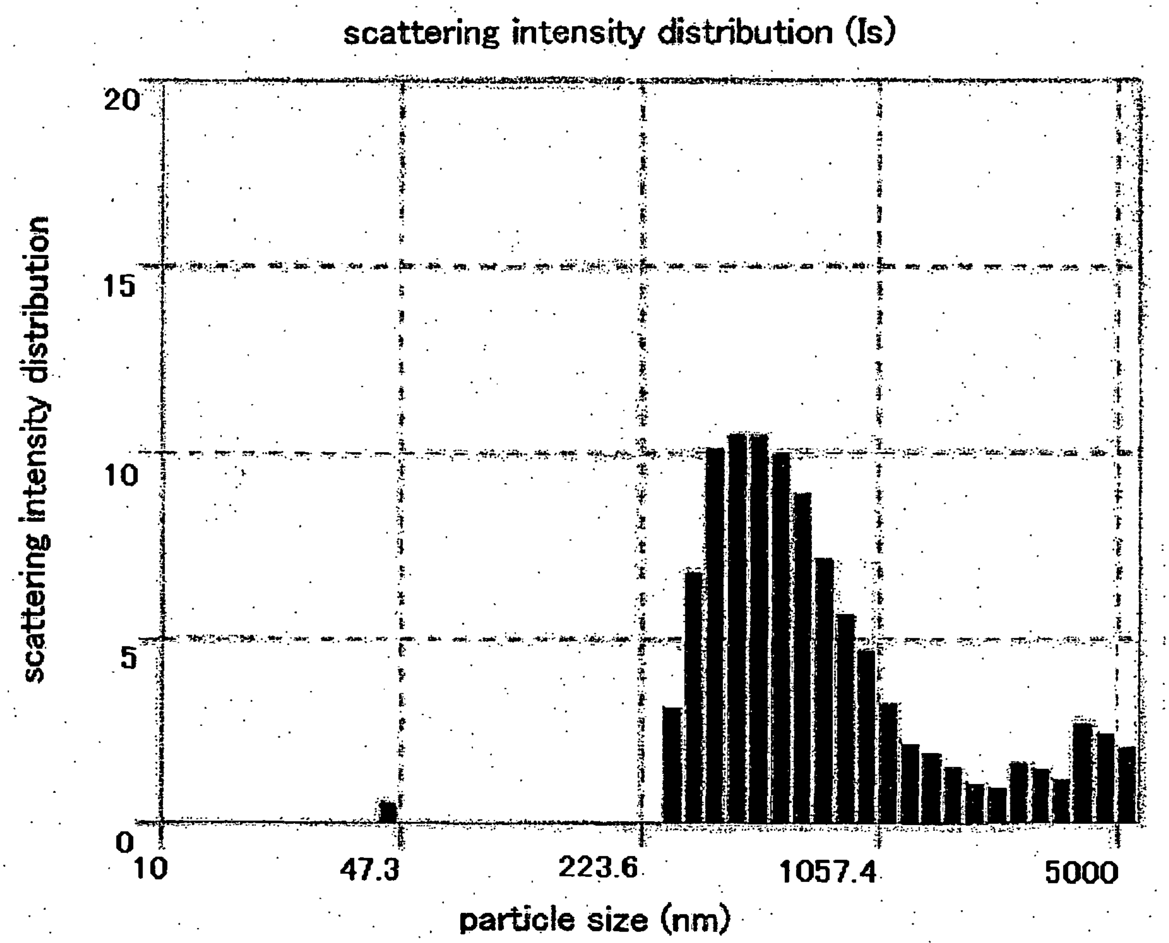
scattering intensity distribution (Is)
scattering intensity distribution
20
15
10
5
0
10
47.3
223.6
1057.4
5000
particle size (nm)

FIG. 12

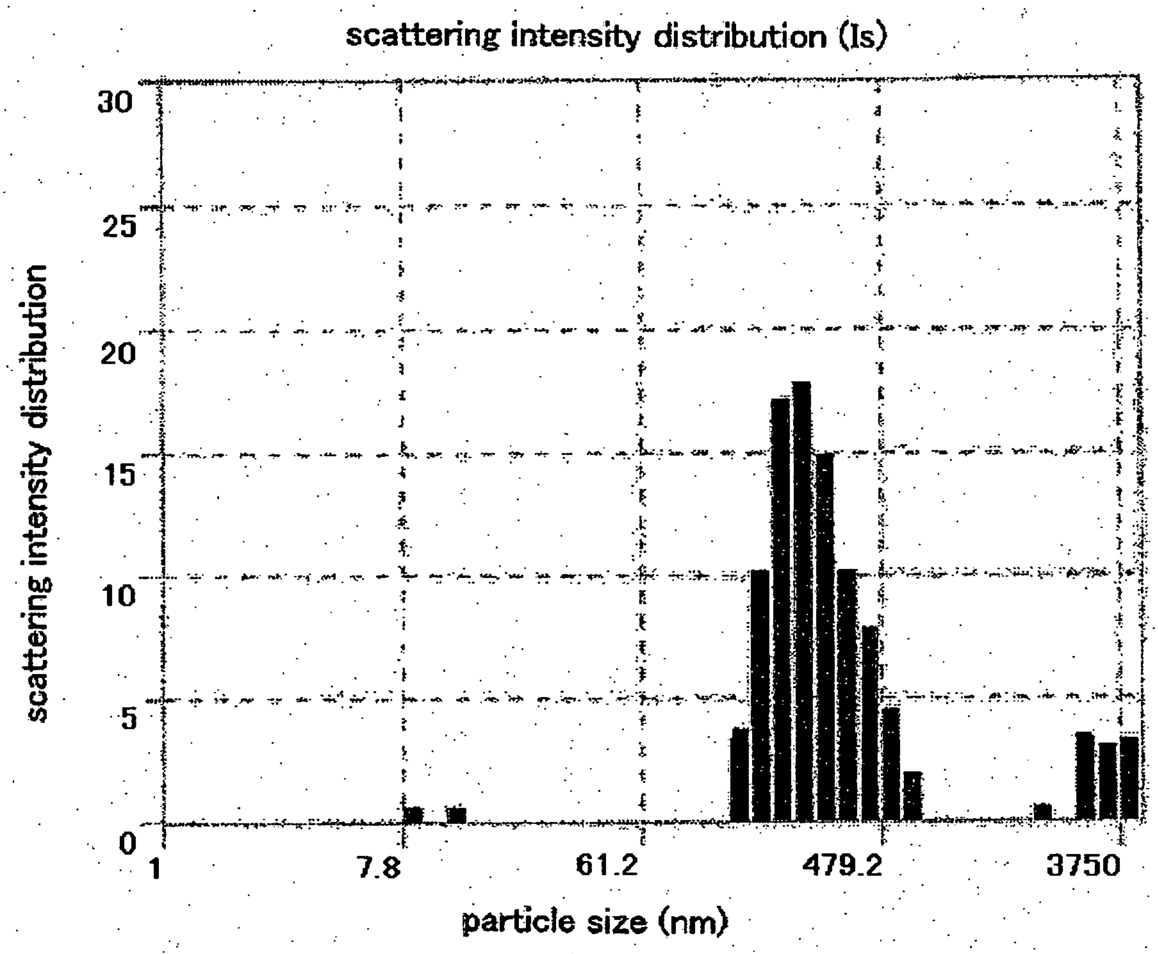
scattering intensity distribution (Is)
scattering intensity distribution
30
25
20
15
10
5
0
1
7.8
61.2
479.2
3750
particle size (nm)

FIG. 13

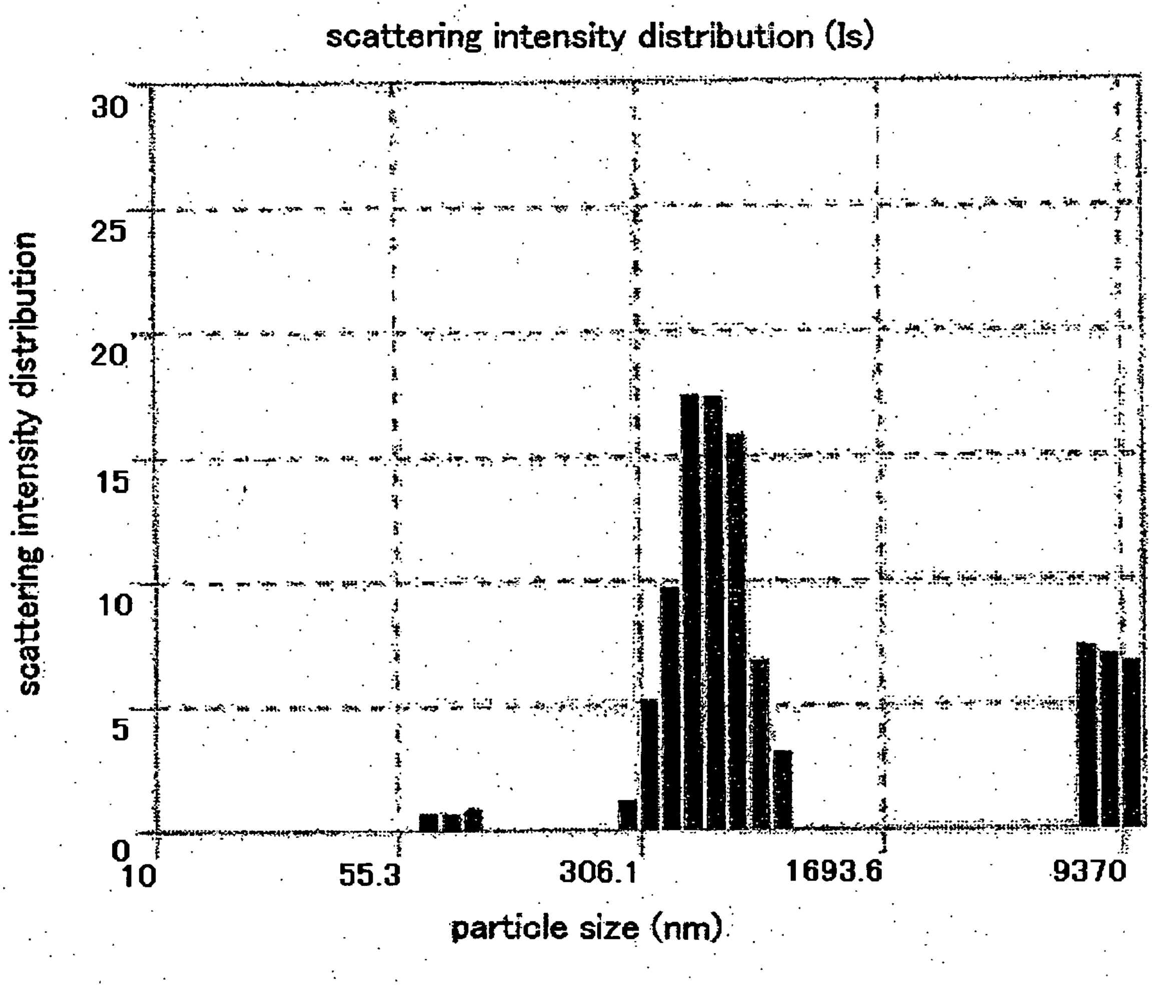
scattering intensity distribution (Is)
scattering intensity distribution
30
25
20
15
10
5
0
10
55.3
306.1
1693.6
9370
particle size (nm)

FIG. 14

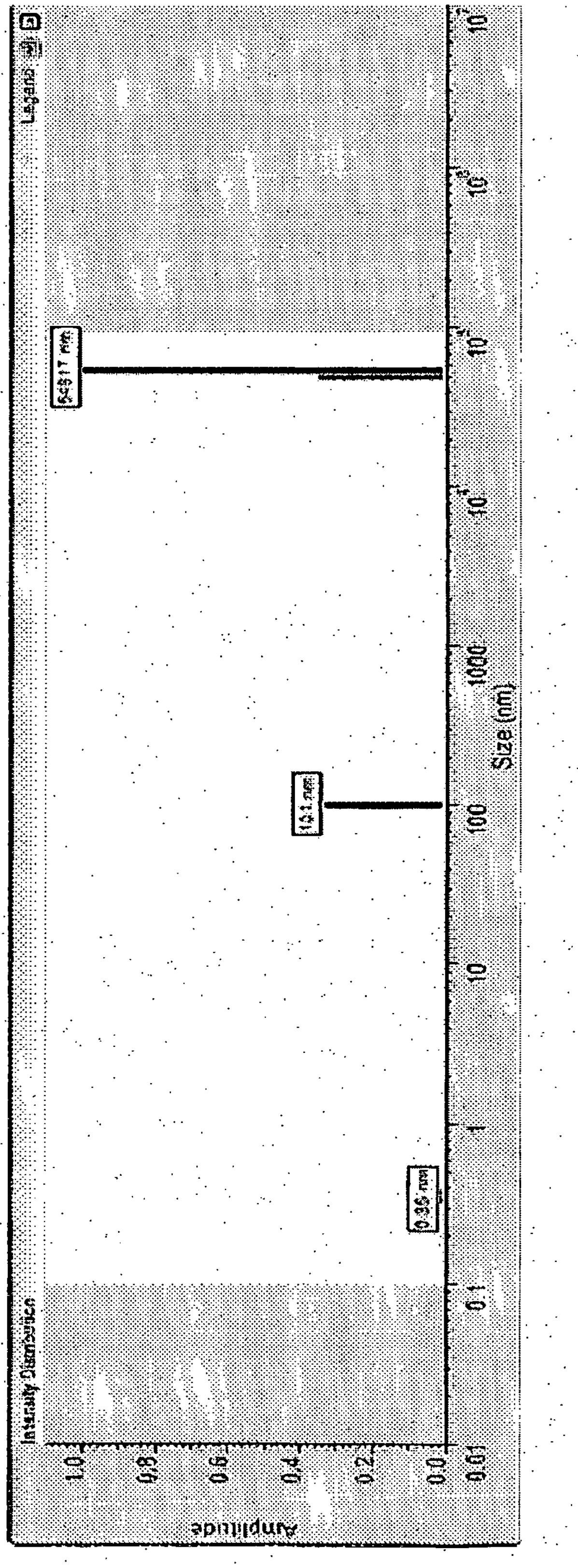
Intensity Distribution
Legend
Amplitude
Size (nm)
0.01
0.1
1
10
100
1000
1.0
0.8
0.6
0.4
0.2
0.0

FIG. 15

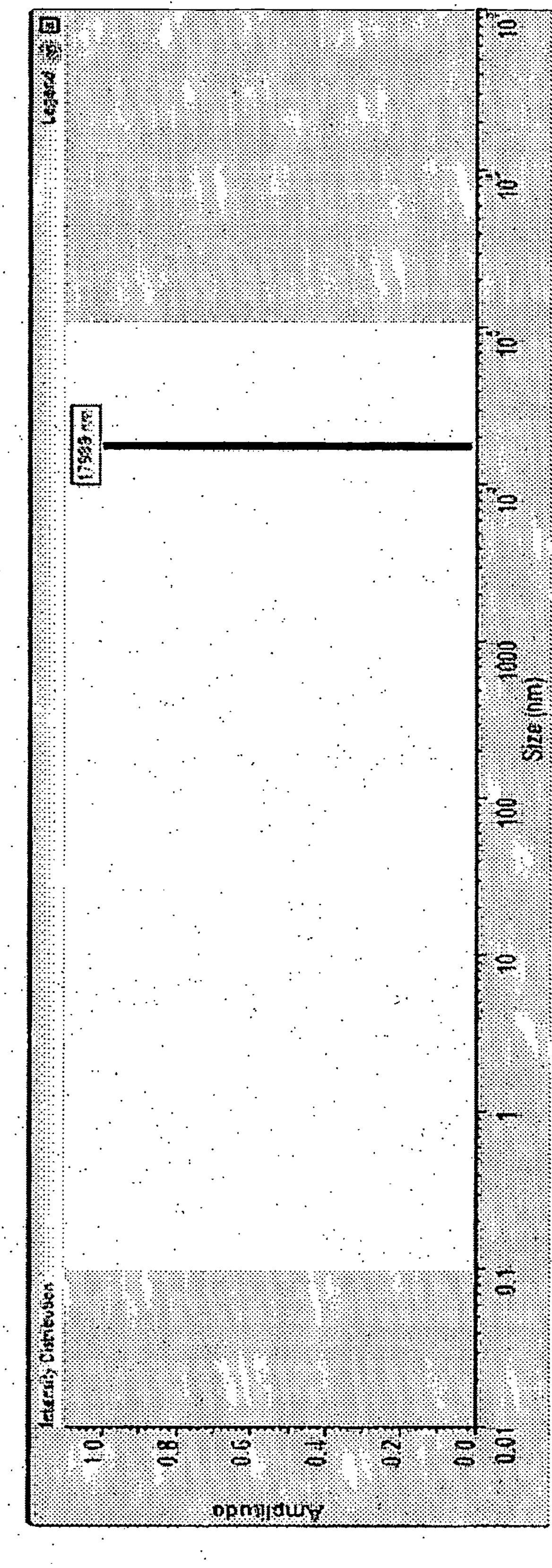
Intensity Distribution
Legend
Amplitude
1.0
0.8
0.6
0.4
0.2
0.0
Size (nm)
0.01
0.1
1
10
100
1000
$10^4$
$10^5$
$10^6$
$10^7$

FIG. 16

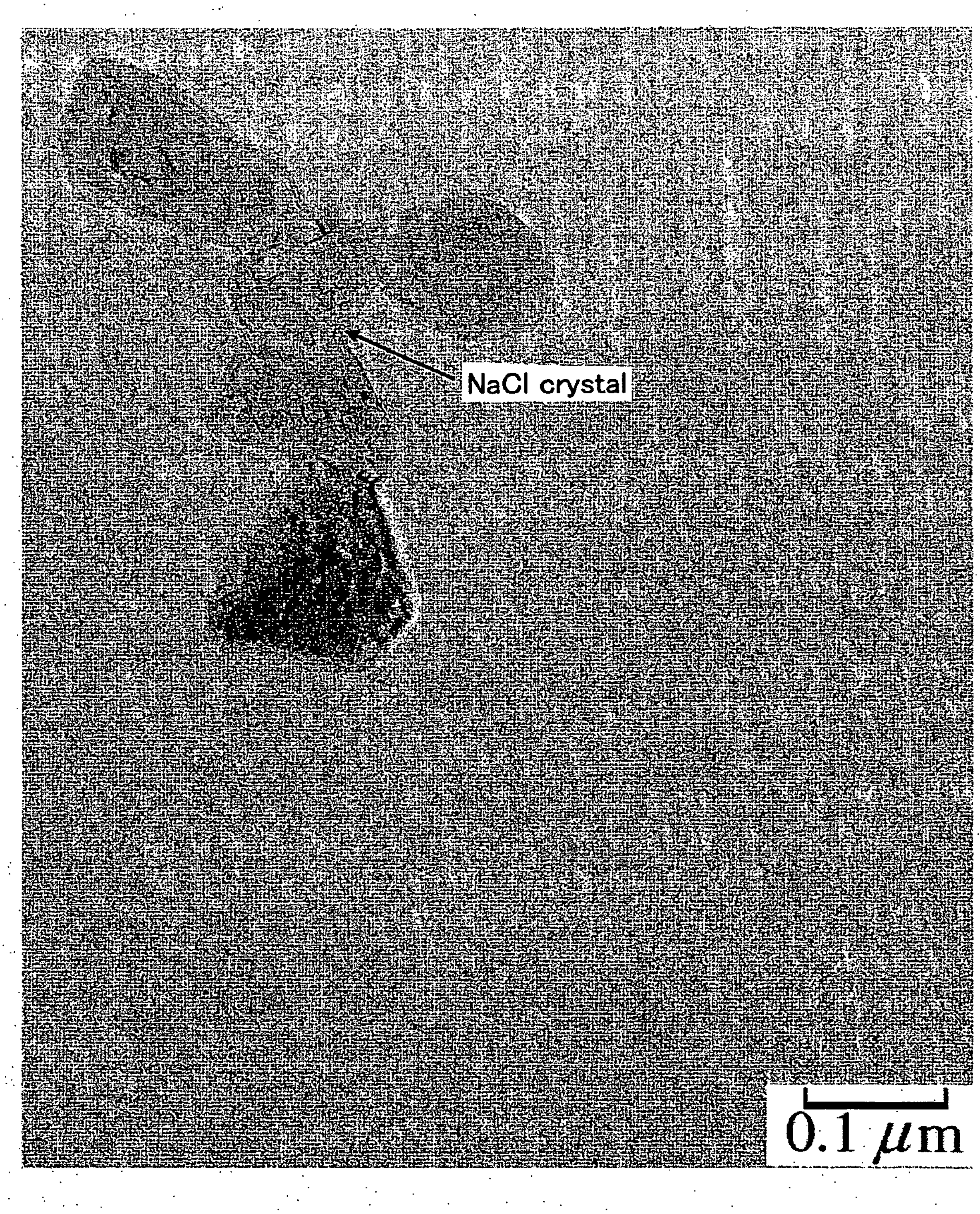

FIG. 17

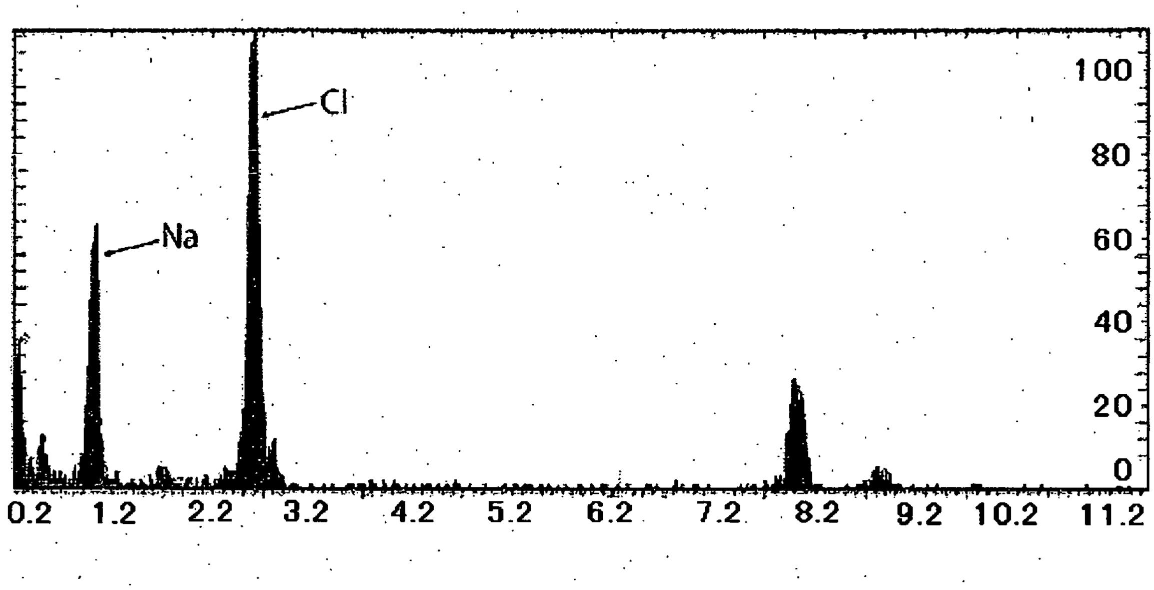

FIG. 18

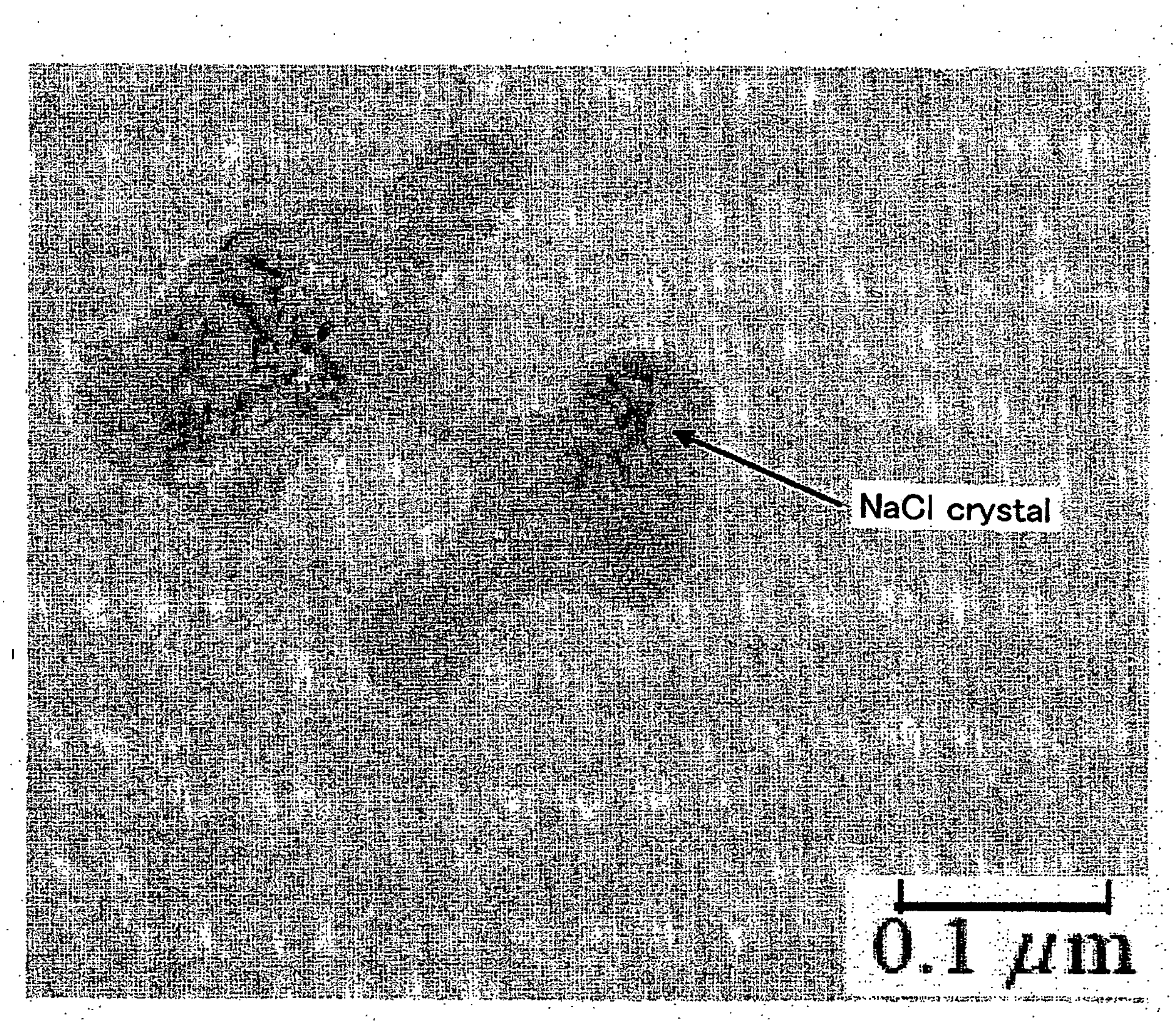

FIG. 19

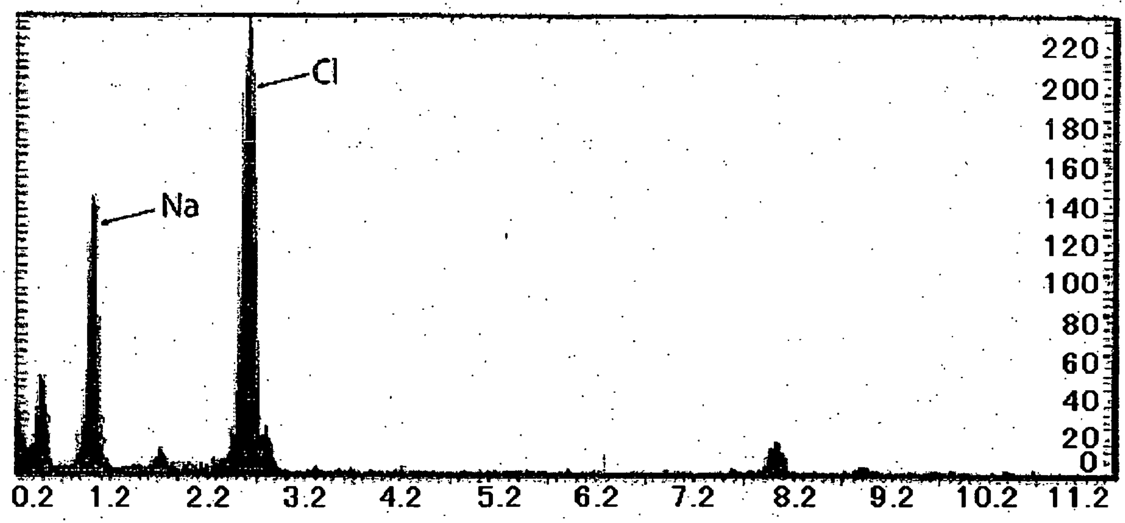
Cl
Na
220
200
180
160
140
120
100
80
60
40
20
0
0.2
1.2
2.2
3.2
4.2
5.2
6.2
7.2
8.2
9.2
10.2
11.2

FIG. 20

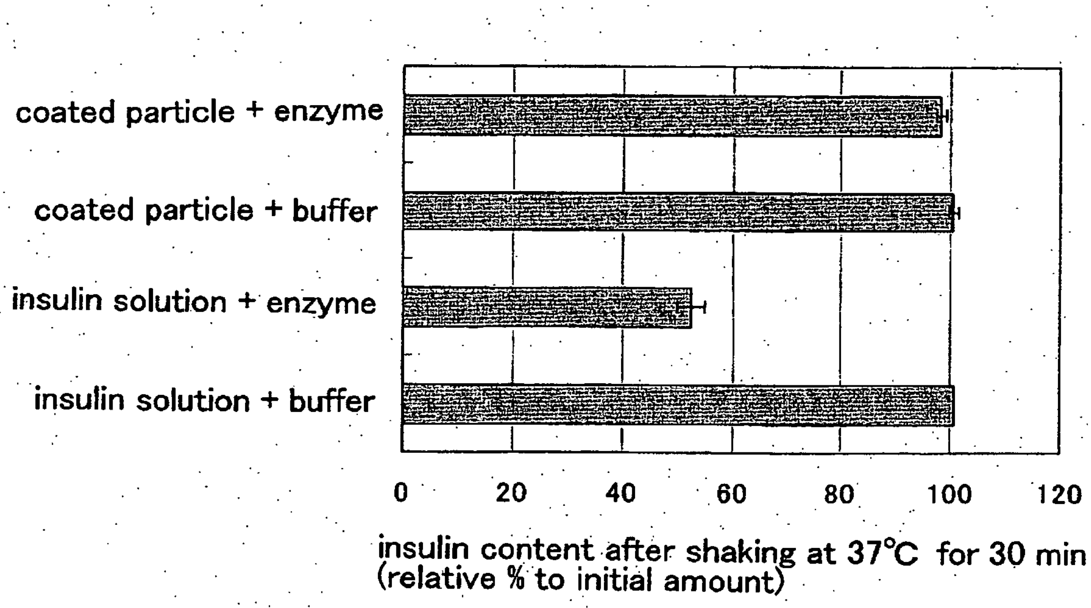
coated particle + enzyme
coated particle + buffer
insulin solution + enzyme
insulin solution + buffer
0
20
40
60
80
100
120
insulin content after shaking at 37°C for 30 min (relative % to initial amount)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040219224 A
- WO 9846732 A
- US 7087246 B
- WO 02072636 A
- WO 9955310 A
- JP 2009231001 A

### Non-patent literature cited in the description

- *Drug Discovery Today,* 2002, vol. 7, 1184-1189
- *J. Control. Rel.,* 2003, vol. 87, 187-198
- *J. Pharm. Sci.,* 2007, vol. 96, 473-483
- *Biomaterials,* 2002, vol. 23, 3193-3201
- *Int J Pharm.,* 2007, vol. 342, 240-249
- *J. Biotech.,* 2004, vol. 113, 151-170
- *Pharm. Res.,* 2005, vol. 22 (9), 1461-1470